# EUROPEAN PATENT SPECIFICATION

(11) **EP 4 103 161 B1**
(45) Date of publication and mention of the grant of the patent: **20.12.2023**
(21) Application number: 21709784.9
(22) Date of filing: 26.02.2021
(51) Int. Cl.: A61K 31/00, A61K 31/4709, A61K 31/472, A61K 31/497, A61K 31/501, A61K 31/517, A61K 31/5377, A61P 1/06

(54) **COMPOSITIONS AND USES THEREOF**
ZUSAMMENSETZUNGEN UND VERWENDUNGEN DAVON
COMPOSITIONS ET LEURS UTILISATIONS

(30) Priority: 28.02.2020 GB 202002926
(43) Date of publication of application: 21.12.2022
(73) Proprietor: BenevolentAI Cambridge Limited, London W1T 5HD (GB)
(72) Inventor: ROBAS, Nicola, London W1T 5HD (GB); BELICH, Monica, London W1T 5HD (GB)
(74) Representative: Appleyard Lees IP LLP
(86) International application number: PCT/GB2021/050498
(87) International publication number: WO 2021/171035

(56) References cited:
- WO-A1-2015/006689
- US-A1- 2017 196 870
- US-A1- 2019 352 277
- JUN KUNITOMO ET AL: "Discovery of 1-[2-Fluoro-4-(1 H -pyrazol-1-yl)phenyl]-5-methoxy-3-(1-pheny l-1 H -pyrazol-5-yl)pyridazin-4(1 H )-one (TAK-063), a Highly Potent, Selective, and Orally Active Phosphodiesterase 10A (PDE10A) Inhibitor", JOURNAL OF MEDICINAL CHEMISTRY, vol. 57, no. 22, 26 November 2014 (2014-11-26), pages 9627-9643, XP055631520, US ISSN: 0022-2623, DOI: 10.1021/jm5013648 cited in the application

## Description

### Technical Field of the Invention

This invention relates to compositions and methods for the prevention, management and/or treatment of inflammatory bowel diseases. More particularly, the invention relates, but not limited, to the treatment of ulcerative colitis.

### Background to the Invention

Inflammatory bowel diseases (IBD) are characterised by chronic uncontrolled inflammation affecting the gastro-intestinal tract and leading to multiple symptoms such as weight loss, abdominal pain, recurrent diarrhoea and bleeding. The prevalence of IBD is around 1 in 1000 people in Europe, with higher prevalence and incidence rates observed in westernized and industrialized countries (Loftus EV, Clinical epidemiology of inflammatory bowel disease: Incidence, prevalence, and environmental influences, Gastroenterology. 2004,126(6):1504-17). Peak incidence occurs in the second to fourth decade of life.

The two major forms are ulcerative colitis (UC) and Crohn's disease (Ramos et al, Mechanisms of Disease: Inflammatory Bowel Diseases., Mayo Clin Proc. 2019, 94(1):155-165). Ulcerative colitis is a chronic lifelong disorder and is characterised by diffuse mucosal inflammation of the colon. The etiology of UC is unknown but the available evidence suggests that both dysregulated innate and adaptive immune pathways contribute to the aberrant intestinal inflammation (Geremia et al, Innate and adaptive immunity in inflammatory bowel disease. Autoimmun Rev. 2014 13(1):3-10)*.*

There is currently no cure for UC. Therapeutic strategies include interventions on lifestyle habits and medical and surgical treatments. Pharmacological management includes corticosteroids, immunosuppressant agents and anti-tumor necrosis factor (TNF)-α biologics (Baumgart et al, Inflammatory bowel disease: clinical aspects and established and evolving therapies, Lancet. 2007 369(9573):1641-57).

US 2017196870 A1 discloses a pharmaceutical composition useful as an agent for treating ulcerative colitis in patients receiving a drug therapy of a 5-aminosalicylic acid preparation and/or a corticosteroid preparation.

US 2019352277 A1 discloses piperidine-2,6-dione derivatives and treatment of Crohn's disease.

There therefore remains an urgent need for a treatment for IBD, and in particular UC. It is an object of the present invention to provide for a treatment which could be used in a range of IBD conditions. It would be advantageous, if such a treatment could be used to treat IBD, but not limited to, UC.

### Summary of the Invention

In accordance with an aspect of the present invention, there is provided a composition comprising a PDE10A inhibitor for use in the prevention, management and/or treatment of inflammatory bowel diseases.

In accordance with a related, but alternative, aspect of the present invention, there is provided an inhibitor of PDE10A for use in the prevention, management and/or treatment of inflammatory bowel diseases.

Suitably the prevention, management and/or treatment of inflammatory bowel diseases involves administering to a patient in need thereof a therapeutically effective amount of the PDE10A inhibitor.

In the following description, where reference is made to the invention encompassing a method of treating a disease using a compound, we intend such references to be interpreted as relating to said compounds for use in said methods for treating said disease.

The present invention is defined by the appended claims. Where embodiments described below are outside the scope of the appended claims, said embodiments serve only to illustrate and place into context the present invention.

In a search for novel mechanisms for inhibiting inflammation the inventors have surprisingly and advantageously found that the selective inhibition of PDE10A with a small molecule inhibitor helps to restore cGMP signalling to normal levels in an *in vitro* assay of IL-8 neutrophil activation and therefore this represents an unexpected and promising treatment for inflammatory bowel diseases, and in particular, ulcerative colitis.

Cyclic nucleotide phosphodiesterases (PDEs) are a family of enzymes that catalyse the degradation of the cyclic nucleotide second messengers cyclic adenosine monophosphate (cAMP) and cyclic guanosine monophosphate (cGMP). Intracellular levels of the cAMP and cGMP are regulated by both their rates of synthesis (by adenylate cyclases and guanylate cyclases respectively) and their hydrolysis by phosphodiesterases. By regulating the duration and amplitude the cAMP and cGMP second messenger signals, PDEs play critical regulatory roles in signal transduction.

There are 11 different PDE subtypes (PDE1-11), each encoding PDEs with unique substrate specificities, kinetics, allosteric regulators, tissue-expression profiles, and pharmacological sensitivities. PDE10A is able to hydrolyse both cAMP and cGMP. PDE10A hydrolyzes cAMP with a *K*ₘ of 0.05 µM and cGMP with a *K*ₘ of 3 µM. Although PDE10A has a lower *K*ₘ for cAMP, the *V*ₘₐₓ ratio cGMP/cAMP is 4.7 indicating a higher specific activity for cGMP. Taken together this suggests that PDE10A is a cAMP-inhibited cGMP phosphodiesterase.

In contrast, PDE4 is known to hydrolyse cAMP and therefore inhibitors of PDE4 may increase levels of cAMP without affecting cGMP levels significantly. Such increased levels of cAMP may have an anti-inflammatory effect.

In normal tissue, PDE10A has a restricted expression pattern. High PDE10A RNA levels are detected only in the striatum (caudate nucleus and putamen) of the brain, and the testes (Fujishige K, Kotera J, Michibata H, Yuasa K, Takebayashi S, Okumura K, Omori K. Cloning and characterization of a novel human phosphodiesterase that hydrolyzes both cAMP and cGMP (PDE10A). J Biol Chem. 1999; 274: 18438-18445). To date inhibitors of PDE10A have mainly been investigated for neurological conditions including schizophrenia and Parkinson's disease (Geerts H, Spiros A, Roberts P. Phosphodiesterase 10 inhibitors in clinical development for CNS disorders. Expert Rev Neurother. 2017, 17(6):553-560). PDE10A has not been investigated extensively for inflammation. A search of the literature identified one paper (Garcia AM et al. Targeting PDE10A GAF Domain with Small Molecules: A Way for Allosteric Modulation with Anti-Inflammatory Effects. Molecules. 2017, 22(9)) that described inhibition of LPS-induced nitrite release from the Raw 264.7 macrophage cell line by a PDE10 inhibitor i.e. in a transformed mouse cell line rather than human primary cells. The authors ascribed the effect seen to the cAMP hydrolytic activity of PDE10A rather than its cGMP activity.

The present inventors have surprisingly found that PDE10A inhibitors described herein can provide a beneficial anti-inflammatory effect in a patient suffering from inflammatory bowel diseases, for example ulcerative colitis, through selective inhibition of cGMP hydrolysis and which can increase levels of cGMP and help restore cGMP signalling to normal levels to.

As used herein, the terms "treatment", "treating", "treat" and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment" as used herein, covers any treatment of a disease in a mammal, particularly in a human, and includes: (a) preventing the disease from occurring in a subject which can be predisposed to the disease but has not yet been diagnosed as having it; (b) inhibiting the disease, i.e., arresting or slowing its development; and (c) relieving the disease, i.e., causing regression of the disease.

The term "subject" or "individual" used herein includes any human or nonhuman animal. The term "nonhuman animal" includes all mammals, such as nonhuman primates, sheep, dogs, cats, cows, horses.

The inflammatory bowel diseases may comprise ulcerative colitis or Crohn's disease. Preferably, the inflammatory bowel disease comprises ulcerative colitis.

The present invention provides PDE10A inhibitors for use in the prevention, management and/or treatment of inflammatory bowel disease. Suitably the inflammatory bowel disease is selected from ulcerative colitis and/or Crohn's disease. Suitably the inflammatory bowel disease is ulcerative colitis.

In some embodiments, the PDE10A inhibitor is a small molecule. Any inhibitor of PDE10A may be suitable for use in any one of the aspects of the present invention. PDE10A inhibitors may include, but not limited to, any one or more of the following: PF-02545920, TAK-063, Papaverine, JNJ-42314415, AMG-579, PG-10, BMS-843496 and PDM-042. It will be apparent to the skilled addressee that a combination or one or more of the PDE10A inhibitors may be used so as to provide a suitable therapy.

In some embodiments, the PDE10A inhibitor may be as described in US 2010/0197651 A1, suitably selected from Examples 1-195 described therein.

In such embodiments, the PDE10A inhibitor suitably has the formula (I): 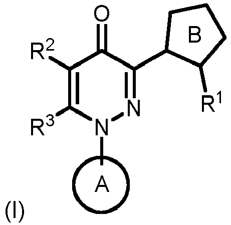 wherein:
R¹ represents a substituent,
R² represents a hydrogen atom or a substituent,
R³ represents a hydrogen atom or a substituent,
ring A represents an aromatic ring which can be substituted, and
ring B represents a 5-membered heteroaromatic ring which can be substituted,
or a salt thereof, or a prodrug thereof.

Suitably R¹ represents a phenyl group which can be substituted by 1 to 5 substituents selected from a halogen atom, a C₁₋₁₀ alkyl group, and a C₁₋₁₀ alkoxy group.

Suitably R² represents a C₁₋₁₀ alkoxy group which can be substituted by one or more substituents selected from a halogen atom, a C₁₋₁₀ alkoxy group, and a C₃₋₇ cycloalkyl group.

Suitably R³ represents a hydrogen atom, or a C₁₋₁₀ alkoxy group.

Suitably ring A represents a benzene ring which can be substituted by 1 to 5 substituents selected from:
(1) a halogen atom,
(2) a C₁₋₁₀ alkyl group which can be substituted by 1 to 3 halogen atoms,
(3) a C₁₋₁₀ alkoxy group which can be substituted by 1 to 3 halogen atoms,
(4) a C₃₋₇ cycloalkyl group,
(5) a halogeno alkylsulfonyloxy group,
(6) a C₃₋₇ cycloalkyl-C₂₋₆ alkynyl group, and
(7) a 4- to 6-membered heterocyclic group containing 0 or 1 oxygen atom, and 1 to 3 nitrogen atoms as heteroatoms which can be substituted by 1 to 4 substituents selected from a halogen atom, a hydroxy group, an oxo group, a C₁₋₁₀ alkoxy-carbonyl group, a C₁₋₁₀ alkoxy group which can be substituted by halogen, and a C₁₋₁₀ alkyl group which can be substituted by halogen.

Suitably ring B represents a pyrazole ring which can be further substituted with 1 to 3 substituents selected from a halogen atom, and a C₁₋₁₀ alkyl group which can be substituted by halogen.

Suitably R¹ represents a phenyl group which can be substituted by 1 to 5 halogen atoms,
R² represents a hydrogen atom or a C1-10 alkoxy group,
R³ represents a hydrogen atom,
ring A represents a benzene ring which is substituted with one 4- to 6-membered heterocyclic group containing 0 or 1 oxygen atom, and 1 to 3 nitrogen atoms as heteroatoms which can be substituted by 1 to 4 substituents selected from a halogen atom, a hydroxy group, an oxo group, halogeno C₁₋₁₀ alkoxy group, a C₁₋₁₀ alkoxy-carbonyl, and a C₁₋₁₀ alkyl group which can be substituted by halogen, and which can be further substituted with 1 or 2 substituents selected from a halogen atom and a C₁₋₁₀ alkoxy group, and
ring B represents a pyrazole ring.

Suitably the 4- to 6-membered heterocyclic group containing 0 or 1 oxygen atom, and 1 to 3 nitrogen atoms as heteroatoms of ring A is a morpholino group, a pyrrolyl group, a dihydropyrrolyl group, a pyrazolyl group, a dihydropyrazolyl group, a piperidyl group, an azetidinyl group, a pyrrolidinyl group, an oxazolidinyl group, an imidazolyl group or an imidazolidinyl group.

In one embodiment, the PDE10A inhibitor is suitably 1-[2-fluoro-4-(3,3,4,4-tetrafluoropyrrolidin-1-yl)phenyl]-5-methoxy-3-(1-phenyl-1H-pyrazol-5-yl)pyridazin-4(1H)-one, or a salt thereof.

In one embodiment, the PDE10A inhibitor is suitably 1-[2-fluoro-4-(2-oxopyrrolidin-1-yl)phenyl]-5-methoxy-3-(1-phenyl-1H-pyrazol-5-yl)pyridazin-4(1H)-one, or a salt thereof.

In one embodiment, the PDE10A inhibitor is suitably 1-[4-(3,4-difluoro-1H-pyrrol-1-yl)-2-fluorophenyl]-5-methoxy-3-(1-phenyl-1H-pyrazol-5-yl)pyridazin-4(1H)-one, or a salt thereof.

In one embodiment, the PDE10A inhibitor is suitably 1-[2-fluoro-4-(1H-pyrazol-1-yl)phenyl]-5-methoxy-3-(1-phenyl-1H-pyrazol-5-yl)pyridazin-4(1H)-one, or a salt thereof.

In one embodiment, the PDE10A inhibitor is suitably 1-[4-(4-chloro-1H-pyrazol-1-yl)-2-fluorophenyl]-5-methoxy-3-(1-phenyl-1H-pyrazol-5-yl)pyridazin-4(1H)-one, or a salt thereof.

In one embodiment, the PDE10A inhibitor is suitably 1-[2-fluoro-4-(2-oxo-1,3-oxazolidin-3-yl)phenyl]-5-methoxy-3-(1-phenyl-1H-pyrazol-5-yl)pyridazin-4(1H)-one, or a salt thereof.

In one embodiment, the PDE10A inhibitor is suitably 3-[1-(2-fluorophenyl)-1H-pyrazol-5-yl]-1-[2-fluoro-4-(1H-pyrazol-1-yl)phenyl]-5-methoxypyridazin-4(1H)-one, or a salt thereof.

In one embodiment, the PDE10A inhibitor is suitably 3-[1-(3-chlorophenyl)-1H-pyrazol-5-yl]-1-[2-fluoro-4-(1H-pyrazol-1-yl)phenyl]-5-methoxypyridazin-4(1H)-one, or a salt thereof.

In one embodiment, the PDE10A inhibitor is suitably 1-[4-(4,4-dimethyl-2-oxopyrrolidin-1-yl)-2-fluorophenyl]-5-methoxy-3-(1-phenyl-1H-pyrazol-5-yl)pyridazin-4(1H)-one, or a salt thereof.

In one embodiment, the PDE10A inhibitor is suitably 1-[4-(5,5-dimethyl-2-oxo-1,3-oxazolidin-3-yl)-2-fluorophenyl]-5-methoxy-3-(1-phenyl-1H-pyrazol-5-yl)pyridazin-4(1H)-one, or a salt thereof.

In one embodiment, the PDE10A inhibitor is suitably 5-methoxy-1-[2-methoxy-4-(1H-pyrazol-1-yl)phenyl]-3-(1-phenyl-1H-pyrazol-5-yl)pyridazin-4(1H)-one, or a salt thereof.

In some embodiments, the PDE10A inhibitor may be as described in US 2013/150344 A1, suitably selected from Examples 1-102.

In such embodiments, the PDE10A inhibitor suitably has the formula (II): 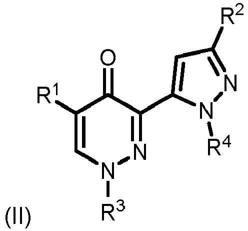 wherein:
R¹ is an optionally substituted C₁₋₆ alkoxy group,
R² is a hydrogen atom or an optionally substituted C₁₋₆ alkyl group,
R³ is: 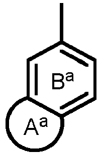 wherein
ring A^{a} is an optionally substituted 5- or 6-membered heterocycle wherein the substituents for
ring A^{a} are optionally bonded to form a ring; and
ring B^{a} is an optionally substituted benzene ring or an optionally substituted pyridine ring,
or R³ is: 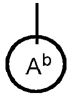 wherein
ring A^{b} is a substituted pyridine ring, an optionally substituted pyrazole ring, an optionally substituted thiazole ring, an optionally substituted pyrazine ring, an optionally substituted pyridazine ring, an optionally substituted pyrimidine ring or an optionally substituted imidazole ring, and
R⁴ is an optionally substituted C₁₋₆ alkyl group or an optionally substituted phenyl group.

Suitably R³ is a substituted pyridine ring or a substituted pyrazole ring.

In one embodiment, the PDE10A inhibitor is suitably 5-{3-[1-(3-Chlorophenyl)-1H-pyrazol-5-yl]-5-methoxy-4-oxopyridazin-1(4H)-yl}-4-fluoro-1,3,3-trimethyl-1,3-dihydro-2H-indol-2-one or a salt thereof.

In one embodiment, the PDE10A inhibitor is suitably 1-(Cyclopropylmethyl)-4-fluoro-5-[5-methoxy-4-oxo-3-(1-phenyl-1H-pyrazol-5-yl)pyridazin-1(4H)-yl]-3,3-dimethyl-1,3-dihydro-2H-indol-2-one or a salt thereof.

In one embodiment, the PDE10A inhibitor is suitably 1-[1-(1-Cyclopropylethyl)-1H-pyrazol-4-yl]-5-methoxy-3-(1-phenyl-1H-pyrazol-5-yl)pyridazin-4(1H)-one or a salt thereof.

In one embodiment, the PDE10A inhibitor is suitably 5-Methoxy-1-[2-methoxy-6-(3,3,4,4-tetrafluoropyrrolidin-1-yl)pyridin-3-yl]-3-(1-phenyl-1H-pyrazol-5-yl)pyridazin-4(1H)-one or a salt thereof.

In one embodiment, the PDE10A inhibitor is suitably 1-[6-(3,4-difluoro-1H-pyrrol-1-yl)-2-methoxypyridin-3-yl]-5-methoxy-3-(1-phenyl-1H-pyrazol-5-yl)pyridazin-4(1H)-one or a salt thereof.

In some embodiments, the PDE10A inhibitor may be as described in US 2014/0178304 A1.

In such embodiments, the PDE10A inhibitor is suitably TAK-063. TAK-063 (balipodect) has the IUPAC formula 1-(2-fluoro-4-pyrazol-1-ylphenyl)-5-methoxy-3-(2-phenylpyrazol-3-yl)pyridazin-4-one (CAS number: 1238697-26-1) and a structure as below:

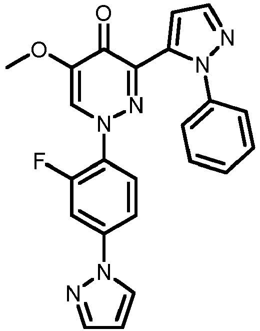

TAK-063 was studied in a phase 2 clinical trial for the treatment of people with schizophrenia. TAK-063 was given at 20 mg once per day but may be reduced to 10 mg once per day if the higher dose was intolerable.

In some embodiments, the PDE10A inhibitor is papaverine. Papaverine has the IUPAC formula 1-[(3,4-dimethoxyphenyl)methyl]-6,7-dimethoxyisoquinoline (CAS number: 58-74-2) and a structure as below:

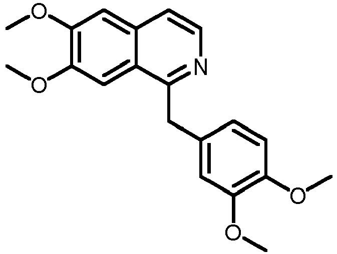

Papaverine is an alkaloid found in opium but not closely related to the other opium alkaloids in its structure or pharmacological actions. It is a direct-acting smooth muscle relaxant used in the treatment of impotence and as a vasodilator, especially for cerebral vasodilation. Papaverine is currently given to patients for the treatment of cerebral and peripheral ischemia associated with arterial spasm and myocardial ischemia complicated by arrhythmias. The dosage is 150 mg every 12 hours orally.

In some embodiments, the PDE10A inhibitor may be as described in WO 2006072828 A2, suitably selected from Examples 1-85 described therein.

In such embodiments, the PDE10A inhibitor suitably has the formula (III): wherein: 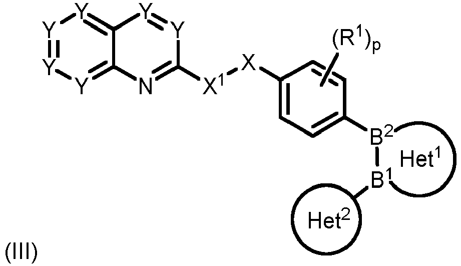
each R¹ is independently selected from a group consisting of hydrogen, halogen, hydroxyl, cyano, C₁ to C₈ alkyl, C₂ to C₈ alkenyl, C₂ to C₈ alkynyl, C₁ to C₈ alkoxy, C₁ to C₈ haloalkyl, C₃ to C₈ cycloalkyl, C₃ to C₈ cycloalkyl-C₁ to C₈ alkyl, 4 to 7 membered heterocycloalkyl, C₁ to C₈ alkylthio, -NR³R³, -C-CF₃, -S(O)ₙR³, C(O)-NR³R³, and C₁ to C₈ alkyl substituted with a heteroatom wherein the heteroatom is selected from a group consisting of nitrogen, oxygen and sulfur and wherein the heteroatom may be further substituted with a substituent selected from a group consisting of hydrogen, C₁ to C₈ alkyl, C₃ to C₈ cycloalkyl, C₂ to C₈ alkenyl, C₂ to C₈ alkynyl, and C₁ to C₈ haloalkyl; each R³ is independently selected from a group consisting of hydrogen, C₁ to C₈ alkyl, C₂ to C₈ alkenyl, C₂ to C₈ alkynyl, C₁ to C₈ haloalkyl, C₃ to C₈ cycloalkyl;
R² is selected from a group consisting of hydrogen, C₁ to C₈ alkyl, C₃ to C₈ cycloalkyl- C₁ to C₈ alkyl, C₂ to C₈ alkenyl, C₂ to C₈ alkynyl, C₁ to C₈ haloalkyl and C₃ to C₈ cycloalkyl;
HET¹ is selected from a group consisting of a monocyclic heteroaryl and a bicyclic heteroaryl, wherein the monocyclic and bicyclic heteroaryl may be optionally substituted with at least one R⁴;
R⁴ is selected from a group consisting of halogen, hydroxyl, cyano, C₁ to C₈ alkyl, C₂ to C₈ alkenyl, C₂ to C₈ alkynyl, C₁ to C₈ alkoxy, C₃ to C₈ cycloalkyl, C₃ to C₈ cycloalkyl-C₁ to C₈ alkyl, C₁ to C₈ alkylthiol and C₁ to C₈ alkyl substituted with a substituent selected from a group consisting of -OR⁸, -NR⁸R⁸, and -SR⁸, wherein R⁸ is independently selected from a group consisting of hydrogen and C₁ to C₈ alkyl;
HET² is a monocyclic or bicyclic heteroaryl, wherein the monocyclic and bicyclic heteroaryl may be substituted with at least one R⁵, with the proviso that HET² is not tetrazole; R⁵ is independently selected from a group consisting of halogen, hydroxyl, cyano, C₁ to C₈ alkyl, C₂ to C₈ alkenyl, C₂ to C₈ alkynyl, C₁ to C₈ alkoxy, C₃ to C₈ cycloalkyl, C₃ to C₈ cycloalkyl-C₁ to C₈ alkyl, C₁ to C₈ alkylthio, -NR⁷R⁷, and C₁ to C₈ haloalkyl;
B¹ and B² are adjacent atoms in Het¹ which are independently selected from a group consisting of carbon and nitrogen;
X and X¹ are each independently selected from a group consisting of oxygen, sulfur, C(R²)₂ and NR²; provided that at least one of X and X¹ is carbon;
Y is selected from a group consisting of carbon and nitrogen, provided that when Y is carbon it is substituted with R⁶; wherein each R⁶ is independently selected from a group consisting of hydrogen, halogen, hydroxyl, cyano, C₁ to C₈ alkyl, C₂ to C₈ alkenyl, C₂ to C₈ alkynyl, C₁ to C₈ alkoxy, C₁ to C₈ cycloalkyl, C₁ to C₈ alkylthio, C₁ to C₈ haloalkyl, NR⁷R⁷ -O-CF₃, -S(O)ₘ- R⁷, and C(O)-NR⁷R⁷ C₁ to C₈ alkyl substituted with a heteroatom wherein the heteroatom is selected from a group consisting of nitrogen, oxygen and sulfur and wherein the heteroatom may be further substituted with a substituent selected from a group consisting of hydrogen, C₁ to C₈ alkyl, C₃ to C₈ cycloalkyl, C₃ to C₈ cycloalkyl-C₁ to C₈ alkyl, C₂ to C₈ alkenyl, C₂ to C₈ alkynyl, and C₁ to C₈ haloalkyl; wherein each R⁷ is independently selected from a group consisting of hydrogen and C₁-C₈ alkyl; p is 1 , 2 or 3; n is 0, 1 or 2; and m is 0, 1 or 2.

Suitably HET¹ is a 5 membered heterocyclic aromatic ring, suitably selected from a group consisting of pyrazole, isoxazole, triazole, oxazole, thiazole and imidazole.

Suitably HET² is selected from a group consisting of 4-pyridyl, 4-pyridazine and isoxazole. Suitably HET² is 4-pyridyl.

Suitably Y is selected from a group consisting of carbon and nitrogen, provided that not more than one Y is nitrogen. Suitably all Ys are carbon.

Suitably X¹ is carbon and X is oxygen.

Suitably, the PDE10A inhibitor is suitably selected from a group consisting of:
2-[-4-(4-Pyridin-4-yl-2H-pyrazol-3-yl)-phenoxymethyl]-quinoline;
2-[4-(2-Methyl-4-pyridin-4-yl-2H-pyrazol-3-yl)-phenoxymethyl]-quinoline;
2-[4-(1-Methyl-4-pyridin-4-yl-1 H-pyrazol-3-yl)-phenoxymethyl]-quinoline;
2-[4-(2-Ethyl-4-pyridin-4-yl-2H-pyrazol-3-yl)-phenoxymethyl]-quinoline;
2-[4-(1-Ethyl-4-pyridin-4-yl-1H-pyrazol-3-yl)-phenoxymethyl]-quinoline;
Dimethyl-(2-{4-pyridin-4-yl-3-[4-(quinolin-2-ylmethoxy)-phenyl]-pyrazol-1-yl}-ethyl)- amine;
Dimethyl-(2-{4-pyridin-4-yl-5-[4-(quinolin-2-ylmethoxy)-phenyl]-pyrazol-1-yl}-ethyl)- amine;
1-{4-Pyridin-4-yl-3-[4-(quinolin-2-ylmethoxy)-phenyl]-pyrazol-1-yl}-propan-2-ol;
1-{4-Pyridin-4-yl-5-[4-(quinolin-2-ylmethoxy)-phenyl]-pyrazol-1-yl}-propan-2-ol;
2-[4-(2-Isopropyl-4-pyridin-4-yl-2H-pyrazol-3-yl)-phenoxymethyl]-quinoline;
2-[4-(4-Pyridin-4yl-isoxazol-5-yl)-phenoxymethyl]-quinoline;
2-[4-(5-Pyridin-4-yl-pyrimidin-4-yl)-phenoxymethyl]-quinoline;
2-[4-(2-Methyl-5-pyridin-4-yl-pyrimidin-4-yl)-phenoxymethyl]-quinoline;
2-[4-(2-Methyl-6-pyridin-4-yl-pyrazolo[1,5-a]pyrimidin-7-yl)-phenoxymethyl]-quinoline;
2-[4-(2-Methyl-6-pyridin-4-yl-[1,2,4]triazolo[1,5-a]pyrimidin-7-yl)-phenoxymethyl]-quinoline 2-[4-(4-Pyridazin-4-yl-2H-pyrazol-3-yl)-phenoxymethyl]-quinoline;
2-[4-(1-Methyl-4-pyridazin-4-yl-1H-pyrazol-3-yl)-phenoxymethyl]-quinoline;
2-[4-(2-Methyl-4-pyridazin-4-yl-2H-pyrazol-3-yl)-phenoxymethyl]-quinoline;
2-[4-(4-Pyrimidin-4yl-2H-pyrazol-3-yl)-phenoxymethyl]-quinoline;
2-[4-(4-Pyridazin-3-yl-2H-pyrazol-3-yl)-phenoxymethyl]-quinoline;
2-{4-[4-(3-Methyl-isoxazol-5-yl)-2H-pyrazol-3-yl]-phenoxymethyl}-quinoline;
2-{4-[2-Methyl-4-(3-methyl-isoxazol-5-yl)-2H-pyrazol-3-yl]-phenoxymethyl}-quinoline;
2-{4-[1-Methyl-4-(3-methyl-isoxazol-5-yl)-1H-pyrazol-3-yl]-phenoxymethyl}-quinoline;
2-{4-[2-Methyl-5-(3-methyl-isoxazol-5-yl)-pyrimidin-4-yl]-phenoxymethyl}-quinoline;
2-[4-(2-Pyridin-4-yl-2H-pyrazol-3-yl)-phenoxymethyl]-quinoline;
2-[4-(3-Methyl-5-pyridin-4-yl[1,2,4]triazol-4-yl)-phenoxymethyl]-quinoline;
2-[4-(1-Methyl-4-pyridin-4-yl-1H-pypazol-3-yl)-phenoxymethyl]-quinoxaline;
7-Chloro-2-[4-(1-methyl-4-pyridin-4-yl-1H-pyrazol-3-yl)-phenoxymethyl]-quinoline hydrogen chloride;
6-Fluoro-2-[4-(1-methyl-4-pyridin-4-yl-1H-pyrazol-3-yl)-phenoxymethyl]-quinoline hydrogen chloride;
2-[2-Fluoro-4-(4-pyridin-4-yl-1H-pyrazol-3-yl)-phenoxymethyl]-quinoline;
2-[2-Fluoro-4-(1-methyl-4-pyridin-4-yl-1 H-pyrazol-3-yl)-phenoxymethyl]-quinoline;
2-[2,3-Difluoro-4-(1-methyl-4-pyridin-4-y!-1 H-pyrazol-3-yl)-phenoxymethyl]-quinoline;
2-[3-Fluoro-4-(4-pyridin-4-yl-1 H-pyrazol-3-yl)-phenoxymethyl]-quinoline;
2-[4-(5-Pyridin-4-yl-1H-pyrazol-4-yl)-phenoxymethyl]-quinoline;
2-[4-(1-Methyl-5-pyridin-4-yl-1 H-pyrazol-4-yl)-phenoxymethyl]-quinoline;
2-[4-(1-Methyl-3-pyridin-4-yl-1H-pyrazol-4-yl)-phenoxymethyl]-quinoline;
2-Methyl-1-{4-pyridin-4-yl-3-[4-(quinolin-2-ylmethoxy)-phenyl]-pyrazol-1-yl}-propan-2- ol;
2-Methyl-1-{4-pyridin-4-yl-5-t4-(quinolin-2-ylmethoxy)-phenyl]-pyrazol-1-yl}-propan-2- ol;
(R)-1-{4-Pyridin-4-yl-3-[4-(quinolin-2-ylmethoxy)-phenyl]-pyrazol-1-yl}-propan-2-ol;
(S)-1-{4-Pyridin-4-yl-3-[4-(quinolin-2-ylmethoxy)-phenyl]-pyrazol-1-yl}-propan-2-ol;
2-t4-(1-Isopropyl-4-pyridin-4-yl-1H-pyrazol-3-yl)-phenoxymethyl]-quinoline;
2-[4-(1-Isobutyl-4-pyridin-4-yl-1H-pyrazol-3-yl)-phenoxymethyl]-quinoline;
2-[4-(1 -Methyl-4-pyridin-4-yl-1 H-pyrazol-3-yl)-phenoxymethyl]-[1.8]naphthyridine;
2-{2-[4-(4-Pyridin-4-yl-2H-pyrazol-3-yl)-phenyl]-ethyl}-quinoline;
2-{2-[4-(1-Methyl-4-pyridin-4-yl-1H-pyrazol-3-yl)-phenyl]-ethyl}-quinoline;
2-{4-[4-(2-Chloro-pyridin-4-yl)-1H-pyrazol-3-yl]-phenoxymethyl}-quinoline;
2-{4-[4-(2-Chloro-pyridin-4-yl)-1-methyl-1H-pyrazol-3-yl]-phenoxymethyl}-quinoline;
2-{4-[1-Methyl-4-(2-methyl-pyridin-4-yl)-1H-pyrazol-3-yl]-phenoxymethyl}-quinoline;
Dimethyl-(4-{1-methyl-3-[4-(quinolin-2-ylmethoxy)-phenyl]-1H-pyrazol-4-yl}-pyr[1,2-a]din-2-yl)-amine;
2-[4-(5-Pyridin-4-yl-pyrazol-1-yl)-phenoxymethyl]-quinoline;
2-[4-(3-Methyl-5-pyridin-4-yl-pyrazol-1-yl)-phenoxymethyl]-quinoline;
2-[2-Chloro-4-(4-pyridin-4-yl-1H-pyrazol-3-yl)-phenoxymethyl]-quinoline;
2-[2-Chloro-4-(1 -methyl-4-pyridin-4-yl-1 H-pyrazol-3-yl)-phenoxymethyl]-quinoline;
2-[4-(4-Pyridin-4-yl-4H-[1,2,4]triazol-3-yl)-phenoxymethyl]-quinoline;
2-[4-(5-Pyridin-4-yl-[1,2,4]triazol-1-yl)-phenoxymethyl]-quinoline;
2-[4-(3-Methyl-5-pyridin-4-yl-[1,2,4]triazol-1-yl)-phenoxymethyl]-quinoline;
2-[4-(2-Pyridin-4-yl-2H-[1,2,4]triazol-3-yl)-phenoxymethyl]-quinoline;
2-[4-(5-Methyl-2-pyridin-4-yl-2H-[1,2,4]triazol-3-yl)-phenoxymethyl]-quinoline;
8-Methoxy-2-[4-(1-methyl-4-pyridin-4-yl-1H-pyrazol-3-yl)-phenoxymethyl]-quinoline;
2-[4-(1-Methyl-4-pyridin-4-yl-1H-pyrazol-3-yl)-phenoxymethyl]-pyrido[1,2-a]pyrimidinone;
2-[4-(1-Methyl-4-pyridin-4-yl-1 H-pyrazol-3-yl)-phenoxymethyl]-quinazoline;
2-[3-Fluoro-4-(1 -methyl-4-pyridin-4-yl-1H-pyrazol-3-yl)-phenoxymethyl]-quinoline;
4-Chloro-2-[4-(1-methyl-4-pyridin-4-yl-1H-pyrazol-3-yl)-phenoxymethyl]-quinoline;
4-Methoxy-2-[4-(1-methyl-4-pyridin-4-yl-1H-pyrazol-3-yl)-phenoxymethyl]-quinoline;
Dimethyl-{2-[4-(1-methyl-4-pyridin-4-yl-1H-pyrazol-3-yl)-phenoxymethyl]-quinolin-4-yl}-amine;
2-[4-(1-Methyl-4-pyridin-4-yl-1H-pyrazol-3-yl)-benzyloxy]-quinoline di-succinic acid;
2-((4-(5-(pyridin-4-yl)oxazol-4-yl)phenoxy)methyl)quinoline;
2-((4-(2-methyl-5-(pyridin-4-yl)oxazol-4-yl)phenoxy)methyl)quinoline;
2-((4-(3-Methyl-4-(pyridin-4-yl)-1H-pyrazol-5-yl)phenoxy)methyl)quinoline;
2-((4-(1,3-dimethyl-4-(pyridin-4-yl)-1H-pyrazol-5-yl)phenoxy)methyl)quinoline;
2-((4-(1,5-dimethyl-4-(pyridin-4-yl)-1H-pyrazol-3-yl)phenoxy)methyl)quinoline;
2-(1-(4-(1-methyl-4-(pyridin-4-yl)-1H-pyrazol-3-yl)phenoxy)ethyl)quinoline;
2-((4-(5-(pyridin-4-yl)-1,2,3-triazol-4-yl)phenoxy)methyl)quinoline;
2-((4-(2-methyl-5-(pyridin-4-yl)-2H-1,2,3-triazol-4-yl)phenoxy)methyl)quinoline;
2-((4-(3-methyl-5-(pyridin-4-yl)-3H-1,2,3-triazol-4-yl)phenoxy)methyl)quinoline;
2-((4-(1-(pyridin-4-yl)-1H-imidazol-2-yl)phenoxy)methyl)quinoline;
2-((4-(5-(pyridin-4-yl)-1H-imidazol-1-yl)phenoxy)methyl)quinoline;
2-((4-(2-methyl-5-(pyridin-4-yl)-1H-imidazol-1-yl)phenoxy)methyl)quinoline;
2-((4-(2-ethyl-5-(pyridin-4-yl)-1H-imidazol-1-yl)phenoxy)nnethyl)quinoline;
2-((4-(2-(pyridin-4-yl)-1H-imidazol-1-yl)phenoxy)methyl)quinoline;
and pharmaceutical acceptable salts thereof.

Suitably, the PDE10A inhibitor is suitably selected from a group consisting of:
2-{4-[-Pyridin-4-yl-2-(2,2,2-trifluoro-ethyl)-2H-pyrazol-3-yl]-phenoxymethyl}-quinoline;
2-{4-[-Pyridin-4-yl-1-(2,2,2-trifluoro-ethyl)-1H-pyrazol-3-yl]-phenoxymethyl}-quinoline;
2-{3-Fluoro-4-[4-pyridin-4-yl-1-(2,2,2-trifluoro-ethyl)-1H-pyrazol-3-yl]-phenoxymethyl}-quinoline;
2-{3-Fluoro-4-[4-pyridin-4-yl-1-(2,2,2-trifluoro-ethyl)-1H-pyrazol-3-yl]-phenoxymethyl}-quinoxaline;
2-{4-[4-Pyridin-4-yl-1-(2,2,2-trifluoro-ethyl)-1H-pyrazol-3-yl]-phenoxymethyl}-quinoxaline; and pharmaceutical acceptable salts thereof.

In some embodiments, the PDE10A inhibitor may be as described in WO 2007077490 A2, suitably selected from Examples 1-37 described therein.

In such embodiments, the PDE10A inhibitor suitably has the formula (IV): wherein: 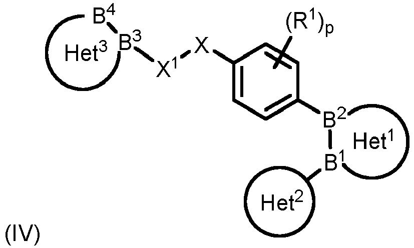
HET¹ is selected from the group consisting of a monocyclic heteroaryl and a, bicyclic heteroaryl, wherein said HET¹ may optionally be substituted with at least one R⁴;
HET² is a monocyclic heteroaryl, wherein said HET² may optionally be substituted with at least one R⁵;
HET³ is an 8 or 9 membered bicyclic heteroaryl, wherein said HET³ may optionally be substituted with at least one R⁶.
R¹ is selected from the group consisting of halogen, hydroxyl, cyano, C₁ to C₈ alkyl, C₂ to C₈ alkenyl, C₂ to C₈ alkynyl, C₁ to C₈ alkoxy, C₁ to C₈ haloalkyl, C₃ to C₈ cycloalkyl, C₂ to C₇ heterocycloalkyl, C₁ to C₈ alkylthio, -NR³R³, -OCF₃, -S(O)ₙ-R³, -C(O)-NR³R³, and C₁ to C₈ alkyl substituted with a heteroatom wherein the heteroatom is selected from the group consisting of nitrogen, oxygen and sulfur and wherein the heteroatom may be further substituted with one or more substituents selected from the group consisting of hydrogen, C₁ to C₈ alkyl, C₃ to C₈ cycloalkyl, C₂ to C₈ alkenyl, C₂ to C₈ alkynyl, and C₁ to C₈ haloalkyl;
each R² is independently selected from the group consisting of hydrogen, C₁ to C₈ alkyl, C₃ to C₈ cycloalkyl-C₁ to C8 alkyl, C₂ to C₈ alkenyl, C₂ to C₈ alkynyl, C₂ to C₈ alkenyl, C₁ to C₈ haloalkyl and C₃ to C₈ cycloalkyl;
each R³ is independently selected from the group consisting of hydrogen, C₁ to C₈ alkyl, C₂ to C₈ alkenyl, C₂ to C₈ alkynyl, C₁ to C₈ haloalkyl, C₃ to C₈ cycloalkyl;
each R⁴ is independently selected from the group consisting of halogen, hydroxyl, cyano, C₁ to C₈ alkyl, C₂ to C₈ alkenyl, C₂ to C₈ alkynyl, C₁ to C₈ alkoxy, C₃ to C₈ cycloalkyl, C₁ to C₈ alkylthio, C₁ to C₈ haloalkyl and C₁ to C₈ alkyl substituted with one or more substituents selected from the group consisting of -OR⁸, -NR⁸R⁸, and -SR⁸;
R⁵ is independently selected from the group consisting of halogen, hydroxyl, cyano, -NR⁸R⁸, C₁ to C₈ alkyl, C₂ to C₈ alkenyl, C₂ to C₈ alkynyl, C₁ to C₈ alkoxy, C₃ to C₈ cycloalkyl, C₁ to C₈ alkylthio, and C₁ to C₈ haloalkyl;
B¹ and B² are adjacent atoms in Het¹ which are independently selected from the group consisting of carbon and nitrogen;
B³ and B⁴ are adjacent atoms in Het³ wherein B³ is carbon and B⁴ is nitrogen;
X and X¹ are each independently selected from the group consisting of oxygen, sulfur, - C(R²)₂ and -NR₂, provided that at least one of X or X¹ is -C(R²)₂;
wherein each R⁶ is independently selected from the group consisting of halogen, hydroxyl, cyano, C₁ to C₈ alkyl, C₂ to C₈ alkenyl, C₂ to C₈ alkynyl, C₁ to C₈ alkoxy, C₁ to C₈ cycloalkyl, C₁ to C₈ alkylthio, C₃ to C₈ haloalkyl, NR⁷R⁷, -O-CF₃, -S(O)ₘ-R⁷, and -C(O)NR⁷R⁷, C₁ to C₈ alkyl substituted with a heteroatom wherein the heteroatom is selected from the group consisting of nitrogen, oxygen and sulfur and wherein the heteroatom may be further substituted with a substituent selected from the group consisting of hydrogen, C₁ to C₈ alkyl, C₁ to C₈ cycloalkyl, C₂ to C₈ alkenyl, C₂ to C₈ alkynyl, and C₁ to C₈ haloalkyl; or two R⁶s together with the atoms which they are attached may optionally form a C₄ to C₁₀ cycloalkyl, C₄ to C₁₀ cycloalkenyl, (4-10 membered) heterocycloalkly or (4-10 membered) heterocycloalkenyl ring;
wherein each R⁷ is independently selected from the group consisting of hydrogen and C₁-C₈ alkyl;
wherein each R⁸ is independently selected from the group consisting of hydrogen, C₁ to C₈ alkyl, C₂ to C₈ alkenyl and C₂ to C₈ alkynyl; n = 0, 1 or 2; m = 0, 1 or 2; and p=0, 1 , 2, 3 or 4.

Suitably HET¹ is a 5 membered heteroaryl, suitably selected from the group consisting of pyrazole, isoxazolyl, triazolyl, oxazolyl, thiazolyl and imidazolyl.

Suitably HET² is selected from the group consisting of 4-pyridyl, 4-pyridazinyl and isoxazolyl, suitably 4-pyridyl.

Suitably, the PDE10A inhibitor is suitably selected from a group consisting of:
1 -Methyl-2-[4-(4-pyridin-4-yl-1 H-pyrazol-3-yl)-phenoxymethyl]-1 H-benzoimidazole;
2-[4-(1-Ethyl-4-pyridin-4-yl-1H-pyrazol-3-yl)-phenoxymethyl]-1-methyl-1H-benzoimidazole; 1-{3-[4-(1-Methyl-1H-benzoimidazol-2-ylmethoxy)-phenyl]-4-pyridin-4-yl-pyrazol-1-yl}-propan-2-ol;
1-Methyl-2-[4-(4-pyridin-4-yl-isoxazol-5-yl)-phenoxymethyl]-1H-benzoimidazole;
1-Methyl-2-[4-(1-methyl-4-pyridin-4-yl-1H-pyrazol-3-yl)-phenoxymethyl]-1H-benzoimidazole;
1-Methyl-2-[4-(2-methyl-4-pyridin-4-yl-2H-pyrazol-3-yl)-phenoxymethyl]-1H-benzoimidazole;
1-Fluoromethyl-2-[4-(1-methyl-4-pyridin-4-yl-1H-pyrazol-3-yl)-phenoxymethyl]-1H-benzoimidazole;
1-Isopropyl-2-[4-(1-methyl-4-pyridin-4-yl-1H-pyrazol-3-yl)-phenoxymethyl]-1H-benzoimidazole;
1-Cyclopropyl-2-(4-(1-methyl-4-pyridin-4-yl-1H-pyrazol-3-yl)-phenoxymethyl]-1H-benzoimidazole;
1-(2-Methoxy-ethyl)-2-[4-(1-methyl-4-pyridin-4-yl-1H-pyrazol-3-yl)-phenoxymethyl]-1H-benzoimidazole;
2-[4-(1-Methyl-4-pyridin-4-yl-1H-pyrazol-3-yl)-phenoxymethyl]-imidazo[1,2-a]pyridine;
2-[4-(2-Methyl-4-pyridin-4-yl-2H-pyrazol-3-yl)-phenoxymethyl]-imidazo[1,2-a]pyridine;
2-[4-(1-Methyl-4-pyridin-4-yl-1H-pyrazol-3-yl)-phenoxymethyl]-[1,2,4]triazolo[1,5-a]-pyridine;
2-{4-[4-Pyridin-4-yl-1-(2,2,2-trifluoro-ethyl)-1H-pyrazol-3-yl]-phenoxymethyl}-[1,2,4]triazolo[1,5-a]pyridine;
2-{4-[4-Pyridin-4-yl-1-(2,2,2-trifluoro-ethyl)-1H-pyrazol-3-y)]-phenoxymethyl}-imidazo[1,2-a]pyridine;
1-Methyl-2-{4-[4-pyridin-4-yl-1-(2,2,2-trifluoro-ethyl)-1H-pyrazol-3-yl]-phenoxymethyl}-1H-benzoimidazole;
1-Fluoromethyl-2-{4-[4-pyridin-4-yl-1-(2,2,2-trifluoro-ethyl)-1H-pyrazol-3-yl]-phenoxymethyl}-1H-benzoimidazole;
1-Methyl-2-[4-(1-methyl-4-pyridin-4-yl-1H-pyrazol-3-yl)-phenoxymethyl]-1H-imidazo[4,5-b]pyridine;
1-Methyl-2-[4-(1-methyl-4-pyridin-4-yl-1H-pyrazol-3-yl)-phenoxymethyl]-1H-imidazo[4,5-c]pyridine;
5,6-Difluoro-1-methyl-2-[4-(1-methyl-4-pyridin-4-yl-1 H-pyrazol-3-yl)-phenoxymethyl]-1H-benzoimidazole;
2-[4-(1-Methyl-4-pyridin-4-yl-1H-pyrazol-3-yl)-phenoxymethyl]-benzothiazole;
2-{4-[4-Pyridin-4-yl-1-(2,2,2-trifluoro-ethyl)-1H-pyrazol-3-yl]-phenoxymethyl}-benzothiazole;
2-[4-(1-Methyl-4-pyridin-4-yl-1H-pyrazol-3-yl)-phenoxymethyl]-5,6-dihydro-4H- imidazo[4,5,1-ij]quinoline;
3- Methyl-2-[4-(1-methyl-4-pyridin-4-yl-1H-pyrazol-3-yl)-phenoxymethyl]-imidazo[1,2-a]pyridine;
2-[4-(1-Methyl-4-pyridin-4-yl-1H-pyrazol-3-yl)-phenoxymethyl]-1-(2,2,2-trifluoro-ethyl)-1H-benzoimidazole;
1-Methyl-2-[4-(5-pyridin-4-yl-pyrazol-1 -yl)-phenoxymethyl]-1H-benzoimidazole;
1-Methyl-2-{2-[4-(1-methyl-4-pyridin-4-yl-1H-pyrazol-3-yl)-phenyl]-ethyl}-1H-benzoimidazole;
1 -Methyl-2-[4-(4-pyridin-4-yl-4H-[1 ,2,4]triazol-3-yl)-phenoxymethyl]-1H-benzoimidazole;
2-Methyl-7-[4-(1-methyl-4-pyridin-4-yl-1H-pyrazol-3-yl)-phenoxymethyl]-thiazolo[3,2-a]pyrimidin-5-one;
7-[4-(1-Methyl-4-pyridin-4-yl-1H-pyrazol-3-yl)-phenoxymethyl]-thiazolo[3,2-a]pyrimidin-5-one;
2-[3-Fluoro-4-(1-methyl-4-pyridin-4-yl-1H-pyrazol-3-yl)-phenoxymethyl]-1-methyl-1H-benzoimidazole;
6-[4-(1-Methyl-4-pyridin-4-yl-1H-pyrazol-3-yl)-phenoxymethyl]-imidazo[2,1-b]thiazole;
2-((4-(5-(pyridin-4-yl)-1H-imidazol-1-yl)phenoxy)methyl)-1-methyl-1H-benzo[d]imidazole;
2-((4-(5-(pyridin-4-yl)-1H-imidazol-1-yl)phenoxy)methyl)-1H-benzo[d]imidazole;
2-((4-(2-methyl-5-(pyridin-4-yl)-1H-imidazol-1-yl)phenoxy)methyl)-1-methyl-1H-benzo[d]imidazole;
2-((4-(2-ethyl-5-(pyridin-4-yl)-1H-imidazol-1-yl)phenoxy)methyl)-1-methyl-1H-benzo[d]imidazole;
2-((4-(2-(pyridin-4-yl)-1H-imidazol-1-yl)phenoxy)methyl)-1-methyl-1H-benzo[d]imidazole; and pharmaceutical acceptable salts thereof.

In some embodiments, the PDE10A inhibitor may be as described in WO 2008001182 A1, suitably selected from the Examples described therein.

In such embodiments, the PDE10A inhibitor suitably has the formula (V): wherein: 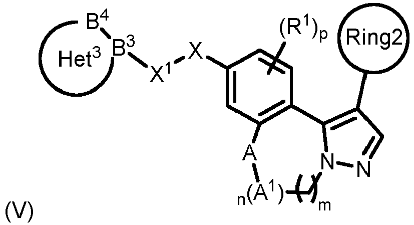
ring 2 is a monocyclic aryl or monocyclic heteroaryl, wherein said ring 2 may optionally be substituted with at least one R⁵;
HET³ is an 8, 9 or 10 membered bicyclic heteroaryl, wherein said HET³ may optionally be substituted with at least one R⁶;
wherein each R¹ is independently selected from the group consisting of halogen, hydroxyl, cyano, C₁ to C₈ alkyl, C₂ to C₈ alkenyl, C₂ to C₈ alkynyl, C₁ to C₈ alkoxy, C₁ to C₈ haloalkyl, C₃ to C₈ cycloalkyl, C₃ to C₇ heterocycloalkyl, C₁ to C₈ alkylthio, -NR³R³, C₁ to C₈ haloalkyloxy, - S(O)ᵣ-R³, -C(O)-NR³R³, and C₁ to C₈ alkyl substituted with a heteroatom wherein the heteroatom is selected from the group consisting of nitrogen, oxygen and sulfur and wherein the heteroatom may be further substituted with one or more substituents selected from the group consisting of hydrogen, C₁ to C₈ alkyl, C₃ to C₈ cycloalkyl, C₂ to C₈ alkenyl, C₂ to C₈ alkynyl, and C₁ to C₈ haloalkyl;
each R³ is independently selected from the group consisting of hydrogen, C₁ to C₈ alkyl, C₂ to C₈ alkenyl, C₂ to C₈ alkynyl, C₁ to C₈ haloalkyl, C₃ to C₈ cycloalkyl;
each R⁵ is independently selected from the group consisting of halogen, hydroxyl, cyano, - NR⁸R⁸, C₁ to C₈ alkyl, C₂ to C₈ alkenyl, C₂ to C₈ alkynyl, C₁ to C₈ alkoxy, C₃ to C₈ cycloalkyl, C₁ to C₈ alkylthio, and C₁ to C₈ haloalkyl;
B³ and B⁴ are adjacent atoms in Het³ wherein B³ is carbon and B⁴ is nitrogen;
X and X¹ are each independently selected from the group consisting of oxygen, sulfur, C(R²)₂ and NR², provided that at least one of X or X¹ is C(R²)₂;
A and A¹ are each independently selected from the group consisting of oxygen, S(O)ₙ C(R⁹)₂ and NR⁹, provided that provided that at least one of A or A¹ is C(R⁹)₂;
wherein each R² is independently selected from the group consisting of hydrogen, C₁ to C₈ alkyl, C₃ to C₈ cycloalkyl-C₁ to C₈ alkyl, C₂ to C₈ alkynyl, C₂ to C₈ alkenyl, C₁ to C₈ haloalkyl and C₃ to C₈ cycloalkyl;
wherein each R⁶ is independently selected from the group consisting of halogen, hydroxyl, cyano, C₁ to C₈ alkyl, C₂ to C₈ alkenyl, C₂ to C₈ alkynyl, C₁ to C₈ alkoxy, C₁ to C₈ haloalkyl, C₁ to C₈ alkylthio, C₃ to C₈ cycloalkyl, -NR⁷R⁷, C₁ to C₈ haloalkyloxy, -S(O)ᵣ-R⁷, -C(O)NR⁷R⁷, C₁ to C₈ alkyl substituted with a heteroatom wherein the heteroatom is selected from the group consisting of nitrogen, oxygen and sulfur and wherein the heteroatom may be further substituted with a substituent selected from the group consisting of hydrogen, C₁ to C₈ alkyl, C₁ to C₈ cycloalkyl, C₂ to C₈ alkenyl, C₂ to C₈ alkynyl, and C₁ to C₈ haloalkyl; or two R⁶s together with the atoms which they are attached may optionally form a C₄ to C₁₀ cycloalkyl, C₄ to C₁₀ cycloalkenyl, (4-10 membered) heterocycloalkly or (4-10 membered) heterocycloalkenyl ring;
wherein each R⁷ is independently selected from the group consisting of hydrogen and C₁-C₈ alkyl;
wherein each R⁸ is independently selected from the group consisting of hydrogen, C₁ to C₈ alkyl, C₂ to C₈ alkenyl and C₂ to C₈ alkynyl;
wherein each R⁹ is independently selected from the group consisting of hydrogen, C₁ to C₈ alkyl, C₃ to C₈ cycloalkyl-C₁ to C₈ alkyl, C₂ to C₈ alkynyl, C₂ to C₈ alkenyl, C₁ to C₈ haloalkyl and C₃ to C₈ cycloalkyl; n = 0 or 1 ; m = 1 or 2, p = 0, 1 , 2 or 3; and r = 0, 1 or 2.

Suitably HET³ is selected from the group consisting of:

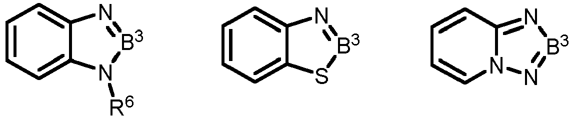

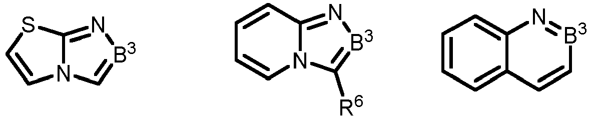

Suitably ring 2 is selected from the group consisting of phenyl, 4-pyridyl, 4-pyridazinyl and isoxazolyl, suitably 4-pyridyl.

Suitably X¹ is C(R²)₂ and X is oxygen.

Suitably m and n are both 1.

Suitably A and A¹ are oxygen and C(R⁹)₂ respectively.

Suitably, the PDE10A inhibitor is suitably selected from a group consisting of:
1-Pyridin-4-yl-8-(quinolin-2-ylmethoxy)-4,5-dihydro-6-oxa-3,3a-diaza-benzo-azulene;
1-Pyridin-4-yl-8-((1-methyl-1H-benzo[d]imidazole-2-y)methanoxy)-4,5-dihydro-6-oxa- 3,3a-diaza-benzo-azulene;
1-Pyridin-4-yl-8-((H-imidazo[1,2-a]pyridin-2-yl)methanoxy)-4,5-dihydro-6-oxa-3,3a-diaza-benzo-azulene;
1-Pyridin-4-yl-8-([1,2,4]triazolo[1,5-a]pyridin-2-ylmethanoxy)-4,5-dihydro-6-oxa-3,3a- diaza-benzo-azulene;
1-Pyridin-4-yl-8-(quinazolin-2-ylmethanoxy)-4,5-dihydro-6-oxa-3,3a-diaza-benzo-azulene;
1-Pyridin-4-yl-8-(imidazo[2,1-b]thiazol-6-ylmethanoxy)-4,5-dihydro-6-oxa-3,3a-diaza- benzo-azulene;
1-Pheny)-4-yl-8-(quinolin-2-ylmethoxy)-4,5-dihydro-6-oxa-3,3a-diaza-benzo-azu!ene;
1-Pyridin-4-yl-8-(quinolin-2-ylmethoxy)-4,5,6-dihydro-3,3a-diaza-benzo-azulene;
and pharmaceutical acceptable salt thereof.

In some embodiments, the PDE10A inhibitor may be as described in US 20140148461 A1, suitably selected from Examples 1-32 described therein.

In such embodiments, the PDE10A inhibitor suitably may be selected from a group consisting of:
4-(1-methyl-3-(4-((1-methyl-1H-imidazol-5-yl)ethynyl)phenyl)-1H-pyrazole-4-yl)pyridine,
2-((4-(1-methyl-4-(pyridin-4-yl)-1H-pyrazol-3-yl)phenyl)ethynyl)pyridine,
5-methyl-2-((4-(1-methyl-4-(pyridin-4-yl)-1H-pyrazol-3-yl)phenyl)ethynyl)pyridine,
4-(1-methyl-3-(4-((1-propyl-1H-pyrazol-4-yl)ethynyl)phenyl)-1H-pyrazole-4-yl)pyridine,
5-fluoro-2-((4-(1-methyl-4-(pyridin-4-yl)-1H-pyrazol-3-yl)phenyl)ethynyl)pyridine,
3-methyl-2-((4-(1-methyl-4-(pyridin-4-yl)-1H-pyrazol-3-yl)phenyl)ethynyl)pyridine,
4-(1-methyl-3-(4-((1-methyl-1H-imidazol-2-yl)ethynyl)phenyl)-1H-pyrazole-4-yl)pyridine,
4-methyl-2-((4-(1-methyl-4-(pyridin-4-yl)-1H-pyrazol-3-yl)phenyl)ethynyl)pyridine,
2-methyl-6-((4-(1-methyl-4-(pyridin-4-yl)-1H-pyrazol-3-yl)phenyl)ethynyl)pyridine,
4-(1-methyl-3-(4-((1-methyl-1H-pyrazol-4-yl)ethynyl)phenyl)-1H-pyrazole-4-yl)pyridine,
4-(3-(4-((1H-pyrazol-4-yl)ethynyl)phenyl)-1-methyl-1H-pyrazole-4-yl)pyridine,
2-((4-(1-methyl-4-(pyridin-4-yl)-1H-pyrazol-3-yl)phenyl)ethynyl)quinoline,
2-[2-[4-[1-methyl-4-(4-pyridyl)pyrazol-3-yl]phenyl]ethynyl]-1,5-naphthyridine,
2-[2-[4-[1-methyl-4-(4-pyridyl)pyrazol-3-yl]phenyl]ethynyl]imidazo[1,2-a]pyridine,
6- [2-[4-[1-methyl-4-(4-pyridyl)pyrazol-3-yl]phenyl]ethynyl]-1H-pyrrolo[2,3-b]pyridine,
4-methyl-2-[2-[4-[1-methyl-4-(4-pyridyl)pyrazol-3-yl]phenyl]ethynyl]quinoline,
2-[2-[4-[1-methyl-4-(4-pyridyl)pyrazol-3-yl]phenyl]ethynyl]thiazole,
2-[2-[4-[1-methyl-4-(4-pyridyl)pyrazol-3-yl]phenyl]ethynyl]quinoxaline,
3-[2-(6-methyl-2-pyridyl)ethynyl]-2-(4-pyridyl)-6,7-dihydro-5H-pyrrolo[1,2-a]imidazole,
2-methoxy-6-[2-[4-[1-methyl-4-(4-pyridyl)pyrazol-3-yl]phenyl]ethynyl]pyridine,
3-methoxy-2-[2-[4-[1-methyl-4-(4-pyridyl)pyrazol-3-yl]phenyl]ethynyl]pyridine,
4-[1-methyl-3-[4-(2-phenylethynyl)phenyl]pyrazol-4-yl]pyridine,
3-[4-[2-(6-methyl-2-pyridyl)ethynyl]phenyl]-2-(4-pyridyl)-6,7-dihydro-5H-pyrrolo[1,2-a]imidazole,
5-[1-methyl-3-[4-[2-(6-methyl-2-pyridyl)ethynyl]phenyl]pyrazol-4-yl]pyrimidine,
2-methyl-6-[2-[4-[1-methyl-4-(1H-pyrazol-4-yl)pyrazol-3-yl]phenyl]ethynyl]pyridine,
2-methyl-6-[2-[4-[2-methyl-4-(4-pyridyl)pyrazol-3-yl]phenyl]ethynyl]pyridine,
2-methyl-6-[2-[4-[1-methyl-4-(2-methylpyrazol-3-yl)pyrazol-3-yl]phenyl]ethynyl]pyridine,
4-[1-methyl-3-[4-[2-(6-methyl-2-pyridyl)ethynyl]phenyl]pyrazol-4-yl]pyridazine,
2-methyl-6-[2-[4-[1-methyl-4-(3-pyridyl)pyrazol-3-yl]phenyl]ethynyl]pyridine,
2-[2-[4-[4-(4-fluorophenyl)-1-methyl-pyrazol-3-yl]phenyl]ethynyl]-6-methyl-pyridine,
2-[2-[4-[4-(4-methoxyphenyl)-1-methyl-pyrazol-3-yl]phenyl]ethynyl]-6-methyl-pyridine,
2-[2-[2-methoxy-4-[1-methyl-4-(4-pyridyl)pyrazol-3-yl]phenyl]ethynyl]-6-methyl-pyridine,
and pharmaceutically acceptable salts thereof.

In some embodiments, the PDE10A inhibitor may be as described in WO 2012133607 A1, suitably selected from the Examples 1-418 described therein.

In such embodiments, the PDE10A inhibitor suitably has the formula (VI): 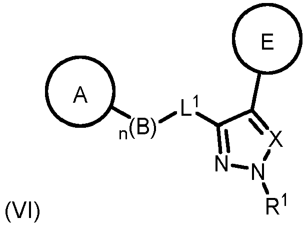 wherein:
ring A is an optionally substituted aromatic heterocycle;
B is C₁₋₆ alkyl, halogen, -O-C₁₋₆ alkyl, optionally substituted cycloalkyl; phenylene or pyridinediyl, thiophenediyl, or -C=C-C-, each optionally substituted with 1 or more of the same or different groups selected from the group consisting of optionally substituted non-aromatic heterocycles;
n is an integer of 0 or 1;
L¹ is -C₁₋₆ alkylene-, -C₁₋₆alkylene-T -, or -T-C₁₋₆ alkylene-;
when n is 0, L¹ is -trimethylene-T- or -tetramethylene-T-;
X is CR⁰ or N;
R¹ is halogen, -OH, -O-C₁₋₆ alkyl, -CN, -C(O)OH and C₁₋₆ alkyl or H, optionally substituted with one or more different groups selected from the group consisting of -C(O)O-C₁₋₆ alkyl;
ring E is an optionally substituted cycloalkyl, optionally substituted aryl, an optionally substituted aromatic heterocycle, or an optionally substituted non-aromatic heterocycle; wherein T is O, S, -NH-, or -(C₁₋₆ alkyl)-; and
R° is H or C₁₋₆ alkyl.

In such embodiments, the PDE10A inhibitor suitably may be selected from a group consisting of:
8-[4-({[1-Methyl-4-(pyridin-4-yl)-1H-pyrazole-3-yl] oxy} methyl) phenyl] quinoline,
1-methyl-5-(1-methyl-3-yl){[4-(3-Methylquinolin-2-yl)phenoxy]methyl}-1H-pyrazole-4-yl)pyridine-2(1H)-one,
1-methyl-5-(1-methyl-3-{[4-(1-Methyl-1H-benzimidazol-4-yl)phenoxy]methyl}-1H-pyrazole-4-yl)pyridine-2(1H)-one,
1-methyl-5-(1-methyl-)3-{[4-(1-Methyl-1H-benzimidazol-4-yl)benzyl]oxy}-1H-pyrazole-4-yl) pyridine-2(1H)-one,
2-(3-{[1-Methyl-4-(pyridin-4-yl)-1H-pyrazole-3-yl]oxy}prop-1-in-1-yl)quinoline,
1-methyl-5-(1-methyl-3-{[4-(3-Methylquinolin-2-yl)benzyl]oxy}-1H-pyrazole-4-yl)piperidin-2-one,
4-(3-{[4-(6-fluoro-3-methylquinolin-)2-yl)benzyl]oxy}-1-methyl-1H-pyrazole-4-yl)-1-methylpyridine-2(1H)-one,
5-(3-{[4-(6-methoxy-3-)Methylquinolin-2-yl)benzyl]oxy}-1-methyl-1H-pyrazole-4-yl)-1-methylpyridine-2(1H)-one,
1-methyl-4-(4-{[1-Methyl-4-(2-methylpyridin-4-yl)-1H-pyrazole-3-yl]methoxy}phenyl)-1H-benzimidazole,
5-(3-{[4-(3-ethylquinolin-2-yl)benzyl]oxy}-1-methyl-1H-pyrazole-4-yl)-1-methylpyridine-2(1H)-one,
3-methyl-2-(4-{[1-methyl-4-(Pyridin-4-yl)-1H-pyrazole-3-yl]methoxy}phenyl)quinoline,
3-methyl-2-[4-({[1-methyl-4-(pyridazine-4-yl)-1H-pyrazole)-3-yl]oxy}methyl)phenyl]quinoline,
2-(4-{[(1,1'-dimethyl-1H,1'H-4,4'-bipyrazole-3-yl)oxy]methyl}phenyl)-3-Methylquinolin,
1-methyl-5-(1-methyl-3-{[4-(quinolin-2-yl)benzyl]oxy}-1H-pyrazole-4-yl)Pylidin-2(1H)-one,
1-ethyl-5-(1-methyl-3-{[4-(3-methylquinolin-2-yl)benzyl]oxy}-1H-pyrazol-4-yl)pyridine-2(1H)-one,
5-(1-methyl-3-{[4-(3-methylquinolin-2-yl)benzyl]oxy}-1H-pyrazol-4-yl)-1-propylpyridine-2(1H)-one,
5-(3-{[4-(6-fluoro-3-methylquinolin-2-yl)benzyl]oxy}-1-methyl-1H-pyrazol-4-yl)-1-methylpyridine-2(1H)-one,
1-methyl-5-(2-methyl-5-{[4-(3-methylquinolin-2-yl)phenoxy]methyl}-2H-1,2,3-Triazole-4-yl)pyridine-2(1H)-one,
3-methyl-2-(4-{[1-methyl-4-(2-methylpyridine-4-yl)-1H-Pyrazole-3-yl]methoxy}phenyl)quinoline,
2-(4-{[4-(2,6-dimethylpyridine-4-yl)-1-methyl-1H-pyrazol-3-yl]methoxy}phenyl)-3-Methylquinolin,
1-methyl-4-(1-methyl-3-{2-[4-(3-methylquinolin-2-yl)phenyl]ethyl}-1H-pyrazol-4-yl)pyridine-2(1H)-one,
1-methyl-4-(1-methyl-3-{[4-(3-methylquinolin-2-yl)phenoxy]methyl}-1H-pyrazol-4-yl)pyridine-2(1H)-one,
1-methyl-4-(4-{[1-methyl-4-(2-methyl-1-oxidepyridine-4-yl)-1H-pyrazol-3-yl]methoxy}phenyl)-1H-benzimidazole,
1-methyl-4-(4-{[1-methyl-4-(pyridazine-4-yl)-1H-pyrazol-3-yl]methoxy}phenyl)-1H-benzimidazole,
1-Methyl-4-(1-methyl-3-{[4-(1-methyl-1H-benzimidazol-4-yl)phenoxy]methyl}-1H-pyrazol-4-yl)pyridine-2(1H)-one,
1'-ethyl-1-methyl-3-{[4-(1-methyl-1H-benzimidazol-4-yl)phenoxy]methyl}-1H,1'H-4,4'-bipyrazole,
and pharmaceutically acceptable salts thereof.

In some embodiments, the PDE10A inhibitor may be as described in US 2013343992 A1, suitably selected from the Examples 1-19 described therein.

In such embodiments, the PDE10A inhibitor suitably has the formula (VII): wherein: 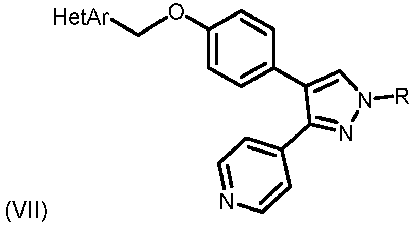
R is selected from the group consisting of hydrogen, C₁₋₆ alkyl, and C₃₋₈ cycloalkyl;
HetAr is a heteroaryl group selected from the group consisting of formula (i) and formula (ii); or 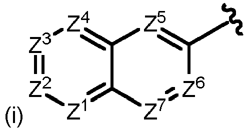
- is absent or a bond; 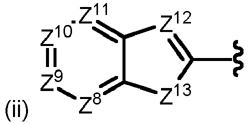
Z¹, Z², Z³, Z⁴, Z⁵, Z⁸, Z⁹, Z¹⁰, Z¹¹, Z¹² are each independently selected from the group consisting of N and CR²;
Z⁶ and Z⁷ are each independently selected from the group consisting of N, NR¹, CR², CHR³, SO₂, and C=O;
Z¹³ is selected from the group consisting of NR¹, CHR³, and O; and
R¹, R², and R³ are each independently selected from the group consisting of hydrogen, halogen, C₁₋₆ alkyl, and C₃₋₈ cycloalkyl;
provided that at least two of Z¹, Z², Z³, Z⁴, Z⁵, Z⁶ and Z⁷ are heteroatoms.

Suitably HetAr is selected from the group consisting of: 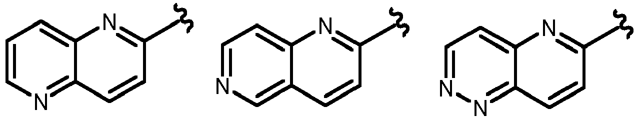 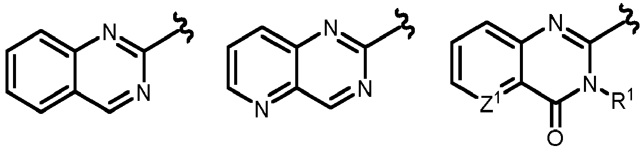 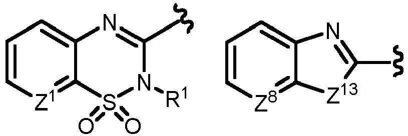 wherein Z¹, Z⁸, Z¹³, and R¹ are as defined above.

In some embodiments, the PDE10A inhibitor is suitably PF-02545920. PF-02545920 (mardepodect, MP-10) has the IUPAC formula 2-[[4-(1-methyl-4-pyridin-4-ylpyrazol-3-yl)phenoxy]methyl]quinolone (CAS number: 898562-94-2) and a structure as below: 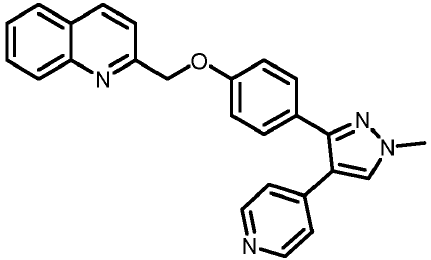 PF-02545920 is a potent and selective cyclic nucleotide PDE10 competitive inhibitor with a reported IC₅₀ value of 1.26 nM. PF-02545920 has been investigated in clinical trials for the treatment of Huntington's Disease. Patients were given 5 or 20 mg of PF-02545920 twice daily.

In some embodiments, the PDE10A inhibitor is JNJ-42314415. JNJ-42314415 has the IUPAC formula 3-[6-(2-methoxyethyl)pyridin-3-yl]-2-methyl-8-morpholin-4-ylimidazo[1,2-a]pyrazine (CAS number: 1334165-90-0) and a structure as below:

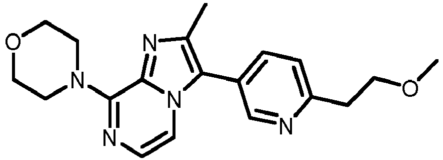

JNJ-42314415 is a potent, selective, centrally active PDE10 inhibitor with Ki of 35 nM for human recombinant PDE10 and demonstrates >100-fold selectivity over the other PDE families.

In some embodiments, the PDE10A inhibitor is AMG-579. AMG-579 has the IUPAC formula 1-[4-[3-[4-(1H-benzimidazole-2-carbonyl)phenoxy]pyrazin-2-yl]piperidin-1-yl]ethanone (CAS number: 1227067-61-9) and a structure as below:

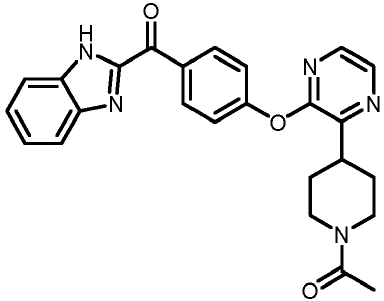

AMG-579 is a potent, selective, CNS penetrant, orally bioavailable PDE10 inhibitor with an IC₅₀ of 0.1 nM. AM-579 displays high selectivity with an IC₅₀ >30 µM against all other PDE isoforms.

In some embodiments, the PDE10A inhibitor is PQ-10. PQ-10 has the IUPAC formula 6,7-dimethoxy-4-[(3R)-3-quinoxalin-2-yloxypyrrolidin-1-yl]quinazoline (CAS number: 927691-21-2) and a structure as below:

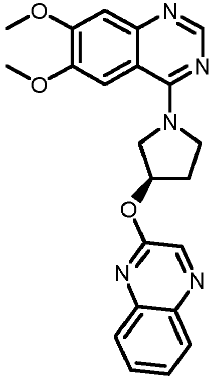

PQ-10 is a potent and selective inhibitor of PDE10 and apparently enhances basic auditory information processing in rats. PQ-10 was given orally to rodents with induced memory deficits. Dosages ranged from 0.1 to 3 mg/kg. PQ-10 was found to be highly brain penetrant and reversed induced memory defects.

In some embodiments, the PDE10A inhibitor is BMS-843496. BMS-843496 has the IUPAC formula 2-((4-chloro-6-((pyridin-3-ylmethyl)amino)pyrimidin-2-yl)amino)-N-ethyl-4-methylthiazole-5-carboxamide (CAS number: 2044975-69-9) and a structure as below:

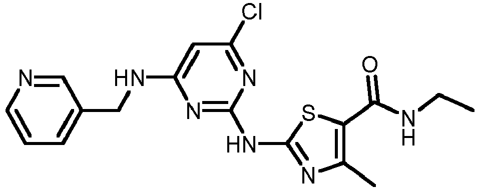

BMS-843496 is a potent and selective PDE10A inhibitor with binding affinity (Kd) of 0.15 nM, an IC₅₀ of 2.11 nM and >100-fold selectivity over other PDE family members. Studies in animals found that administering BMS-843496 was correlated with antipsychotic effects measured using the conditioned avoidance response model.

In some embodiments, the PDE10A inhibitor is PDM-042. PDM-042 has the IUPAC formula (E)-4-(2-(2-(5,8-dimethyl-[1,2,4]triazolo[1,5-a]pyrazin-2-yl)vinyl)-6-(pyrrolidin-1-yl)pyrimidin-4-yl)morpholine (CAS number: ) and a structure as below:

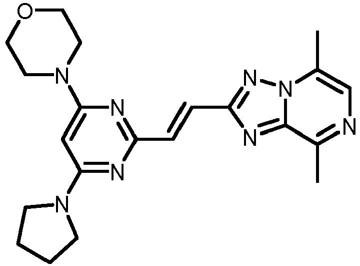

PDM-042 showed potent inhibitory activities for human and rat PDE10 with IC₅₀ values of less than 1 nmol/L and more than 1000-fold selectivity against other phosphodiesterases. In behavioural studies using rat models relevant to schizophrenia, PDM-042 dosed at between 0.1-0.3 mg/kg significantly antagonized MK-801-induced hyperlocomotion without affecting spontaneous locomotor activity and attenuated the conditioned avoidance response.

The PDE10A inhibitor may be selected from PF-02545920 or TAK-063.

The PDE10A inhibitor may be a selective inhibitor of PDE10A. Suitably the PDE10A inhibitor has an IC50 for PDE10A of less than 5 nM. Suitably the PDE10A inhibitor has an IC50 for PDE10A of less than 5 nM and IC50s for the other PDE family members of >1 µM. Suitable assays for measuring the IC50s of the inhibitor against PDE10A and other PDE family members are known in the art.

Suitably the PDE10A inhibitor selectively inhibits cGMP hydrolysis, suitably in the bowel tissue of a patient having inflammatory bowel disease. Suitably the PDE10A inhibitor selectively inhibits cGMP hydrolysis over cAMP hydrolysis.

Suitably the prevention, management and/or treatment of inflammatory bowel diseases of the present invention, provided by the PDE10A inhibitor, involves increasing cGMP signalling in bowel tissue of a patient.

Suitably the prevention, management and/or treatment of inflammatory bowel diseases of the present invention, provided by the PDE10A inhibitor, involves increasing cGMP signalling and cAMP signalling in bowel tissue of a patient.

Suitably the prevention, management and/or treatment of inflammatory bowel diseases of the present invention, provided by the PDE10A inhibitor, involves reducing levels of inflammatory cytokines in bowel tissue of a patient.

For use according to any aspect of the present invention, the therapeutic ingredient(s), such as the inhibitors referred to herein, may be provided as any pharmaceutically acceptable derivative, selected from, but not limited to, any one or more of the following: pharmaceutically acceptable salts, pharmaceutically acceptable solvates, pharmaceutically acceptable enantiomers, pharmaceutically acceptable hydrates, pharmaceutically acceptable polymorphs, pharmaceutically acceptable esters and pharmaceutically acceptable prodrugs.

For use according to any one of the aspects of the present invention, the therapeutic ingredient(s), such as the inhibitors referred to herein may be administered at any suitable pharmacological dose, it being understood that the exact amounts (i.e. the therapeutically effective amounts) will depend upon the nature of the inhibitor and the condition to be treated. For example, suitable doses may comprise a daily dosage of from about 0.1 milligram to about 100 milligrams per kilogram of animal body weight, preferably given as a single daily dose or in divided doses two to six times a day, or in sustained release form.

For most large mammals, the total daily dosage is preferably from about 1 milligram to about 1000 milligrams, more preferably from about 1 milligram to about 350 milligrams, especially from about 1 mg to about 100 mg. In the case of a 70 kg adult human, the total daily dose is preferably in the range of from about 7 milligrams to about 350 milligrams. Typically, such doses may be in the range of from about 50 to 500 mg per day, such as about 240mg or about 100mg per day. This dosage regimen may be adjusted, as known by those skilled in the art, to provide the optimal therapeutic response.

Accordingly, the present invention further provides a use or method as described herein, wherein one or more therapeutic ingredients, including the inhibitor referred to herein, may be comprised in a pharmaceutical formulation, optionally together with one or more pharmaceutically acceptable carriers therefor. The one or more pharmaceutically acceptable carriers of the/each therapeutic ingredient may be the same or different.

Optionally, other therapeutic ingredients may be included in the pharmaceutical formulations of the present invention described herein.

For example, in accordance with any one of the aspects of the present invention described herein, the pharmaceutical formulation may further comprise an inhibitor of inflammatory signalling, including those described below. Accordingly, in accordance with any one of the aspects of the present invention described herein, the PDE10A inhibitor may be for administration separately, sequentially or simultaneously with one or more pharmaceutically active ingredients.

The formulations include compositions suitable for oral, rectal, topical, parenteral, including subcutaneous, intramuscular, and intravenous, ocular (ophthalmic), pulmonary (nasal or buccal inhalation), or nasal administration (such as, for example, in the form of liquid drops or spray), although the most suitable route in any given case will depend on the nature and severity of the conditions being treated and on the nature of the therapeutic ingredient(s), e.g. the nature of the PDE10A inhibitor, and/or other active ingredients present.

In practical use, the therapeutic ingredient(s), such as the inhibitors referred to herein, can be brought into an intimate physical admixture with one or more pharmaceutical carriers according to conventional pharmaceutical formulating techniques. The carrier(s) may take a wide variety of forms depending on the form of preparation desired for administration, e.g., oral or parenteral (including intramuscular and intravenous).

In preparing the formulations/compositions in their dosage form for administration, any of the usual pharmaceutical excipients may be employed, such as, for example, diluents of a solid or liquid nature, flavouring agents, preservatives, colouring agents, and the like. Liquid preparations may be in the form of, for example, suspensions, elixirs and solutions.

Such liquid preparations may comprise one or more of: sucrose as a sweetening agent, methyl and/or propylparabens as preservatives, a dye and flavouring. Oral solid preparations are preferred over oral liquid preparations and are preferably in the form of, for example, powders, hard and soft capsules and tablets. When in solid form, the one or more pharmaceutical carriers may include one or more of: starches, sugars, microcrystalline cellulose, solid or liquid diluents, granulating agents, lubricants, binders, disintegrating agents and the like.

The tablets, pills, capsules, and the like may also contain one or more of: a binder such as gum tragacanth, acacia, corn starch or gelatin; excipients such as dicalcium phosphate; a disintegrating agent such as corn starch, potato starch, alginic acid; a lubricant such as magnesium stearate; and a sweetening agent such as sucrose, lactose or saccharin. When a dosage unit form is a capsule, it may contain, in addition to materials of the above type, comprise a liquid carrier such as a fatty oil.

Because of their ease of administration, tablets and capsules represent an advantageous oral dosage unit form. If desired, tablets may be coated by standard aqueous or non-aqueous techniques. For example, tablets may be coated with shellac, sugar or both.

Such formulations, compositions and preparations will preferably contain at least 0.1% of the therapeutic ingredient(s), such as the inhibitors referred to herein (e.g. the PDE10As inhibitors). The percentage of the therapeutic ingredient(s), such as the inhibitors referred to herein in these formulations/compositions may, of course, be varied and may conveniently be between about 2% to about 60% by weight of the unit dose. The amount of the therapeutic ingredient(s), such as the inhibitors referred to herein in such therapeutically useful compositions is such that an effective dosage (i.e. the therapeutically effective amount) will be obtained.

Various other materials may be present to act as coatings or to modify the physical form of the dosage unit.

For parenteral administration, the pharmaceutical forms include sterile aqueous solutions or dispersions and sterile powders for the extemporaneous preparation of sterile injectable solutions or dispersions. Solutions or suspensions of these active inhibitors can be prepared in water for injections, optionally mixed with a surfactant such as hydroxypropylcellulose. Dispersions can also be prepared in glycerol, liquid polyethylene glycols and mixtures thereof in oils.

Accordingly, the pharmaceutical carrier(s) can be a solvent or dispersion medium containing, for example, water, an alcohol (e.g. ethanol), a polyol (e.g. glycerol, propylene glycol and liquid polyethylene glycols), suitable mixtures thereof, and vegetable oils, and combinations thereof. In all cases, the form must be sterile and must be fluid to enable administration via a syringe. It must be stable under the conditions of manufacture and storage. Under ordinary conditions of storage and use, these preparations contain a preservative to prevent the growth of microorganisms.

In any one of the medical uses of the present invention, "therapeutically effective amount" refers to the amount of the PDE10A inhibitor that, when administered to a subject for preventative or therapeutic treatment, is sufficient to effect the desired treatment (e.g. of the disease). The "therapeutically effective amount" will vary, for example, depending on the inhibitor(s) used, the state of the disease and its severity and the age, weight, etc., of the subject to be treated, as described above.

In any one of the medical uses involving the prevention, management and/or treatment of inflammatory bowel diseases in a subject according to the invention, the PDE10A inhibitor, or formulations thereof as described above may be administered to a subject using any one of the available methods and routes suitable for drug delivery, as described herein.

The PDE10A inhibitor or a formulation thereof, may be administered in a single dose or in multiple doses. A suitable frequency of administration may be at least once per day, every other day, once per week, once every two, three, or four weeks, once every month, two months, or once every three to six months. The PDE10A inhibitor may be administered over a period of at least a week, at least a month, at least three to six months, at least one, two, three, four, or five years, or over the course of the disease, or the lifetime of the subject. Preferably, the subject referred to in any one of the aspects of the present invention is a mammal; humans are preferred. However, both human and veterinary subjects are within the scope of the invention. For veterinary applications, the age of the animal would be scaled from the human situation using the average lifespan for calibration.

The inhibitor may be artificially generated. That is to say that it is not naturally occurring. The inhibitor may however be a naturally occurring molecule whose concentration and formulation in a medicament or pharmaceutical preparation enables it to be used for the prevention, amelioration or treatment of inflammatory bowel diseases, whereas otherwise it would have no or limited efficacy.

In embodiments, the inhibitor comprises a small molecule. The small molecule may be any appropriate organic molecule that inhibits PDE10A.

The inhibitor may comprise an antibody or antibody mixture. Such antibody or antibodies may be polyclonal or may be monoclonal.

The monoclonal antibodies may be obtained by any suitable method. Suitable methods will be known to a person skilled in the art. For example, the monoclonal antibodies may be obtained using hybridoma technology, such as fusing antibody producing cells of antigen-immunised mammals with mammalian myeloma cells to produce a hybridoma cell line. Preferably, the hybridoma cell line is produced by first immunising a mammal with an immunising antigen to produce an immunised mammal. Suitable immunising antigens are as defined above. The mammal may be immunised by any suitable method such as, for example, by intraperitoneal, subcutaneous, intravascular, intramuscular or intrasplenic injection or by oral administration. Preferably, the immunising antigen may be administered as a suspension or solution in a buffer, such as phosphate buffered saline (PBS), optionally with an adjuvant, such as Freund's complete adjuvant. Any suitable mammal may be used such as, for example, mice, rats, rabbits, sheep or goats. It will be appreciated by a person skilled in the art that the mammal should typically be chosen so as to be compatible with the myeloma cells used in the subsequent cell fusion step. Preferably, the immunising antigen is administered to the mammal several times, such as 2, 3, 4 or more times, at 4 to 21 day intervals.

Typically, the antibody producing cells of the immunised mammal may be splenic cells. Preferably, the splenic cells of the immunised mammal may be collected and fused with mammalian myeloma cells. The mammalian myeloma cells may be from any suitable source such as, for example, mice, rats or rabbits. Preferably, the mammalian myeloma cells may be from the same mammalian source as the mammal immunised with the immunising antigen. Preferably, the myeloma cells may be from mice. Preferably, the mammalian myeloma cells are selected so as to have a hypoxyanthine-guanine-phosphoribosyltransferase deficiency (HGPRT⁻) and/or a thymidine kinase deficiency (TK⁻). For example, the mammalian myeloma cells may be mouse P3/NS1/1-Aq4-1 cells. The splenic cells may be fused to the mammalian myeloma cells by any suitable method. Suitable methods will be known to a person skilled in the art. For example, the splenic cells may be fused to the mammalian myeloma cells using electrofusion, optionally in the presence of a fusion promoter such as, for example, polyethylene glycol (PEG) or hemagglutinating virus of Japan (HVJ). Preferably, the splenic cells and the mammalian myeloma cells may be mixed at a ratio of 1:1 to 10:1.

Typically, the fused cells may be cultured and screened to selectively obtain hybridomas. The fused cells may be cultured in any suitable medium. The fused cells may be screened for hybridomas by any suitable method. Suitable methods will be known to a person skilled in the art. For example, the fused cells may be screened for hybridomas using enzyme immunoassay, such as enzyme-linked immunosorbent assay (ELISA), radioimmunoassay (RIA) or surface plasmon resonance (SPR). Preferably, the fused cells may be screened for hybridomas using enzyme-linked immunosorbent assay (ELISA). It will be appreciated by a person skilled in the art that it is the supernatant of the culture of the fused cells that is typically screened. Preferably, the fused cells may be screened for hybridomas by screening for binding to PDE10A. More preferably, the fused cells may be screened for hybridomas by screening for binding to PDE10A, using enzyme-linked immunosorbent assay (ELISA).

The monoclonal antibodies produced by the cultured hybridomas may be obtained by any suitable method. Suitable methods will be known to a person skilled in the art. For example, the monoclonal antibodies produced by the cultured hybridomas may be obtained by centrifugation of the supernatant of the culture.

In certain alternative embodiments, when the monoclonal antibodies are produced by culturing the obtained hybridomas in the abdominal cavities of a suitable mammal such as, for example, a mouse, the obtained hybridomas may be intraperitoneally administered to the mammal such as, for example, mouse. The monoclonal antibodies produced by the culture in the abdominal cavities of a suitable mammal such as, for example, a mouse may then be obtained by collecting the fluid in the peritoneal cavity.

The monoclonal antibodies produced by culturing the obtained hybridomas in a suitable medium or in the abdominal cavities of a suitable mammal such as, for example, a mouse may be used directly or may be purified. Preferably, the monoclonal antibodies may be purified. The monoclonal antibodies may be purified by any suitable method. Suitable methods will be known to a person skilled in the art. For example, the monoclonal antibodies may be purified by ammonium sulphate precipitation, ion exchange chromatography or an anti-IgG antibody column.

If monoclonal antibodies are employed which have binding with PDE10A (or its ligand) then such antibodies may be used in combination (or combined) with small molecule inhibitors of PDE10A.

In certain embodiments, the antibody used in the present invention may be polyclonal. The polyclonal antibodies may be produced by any suitable method. Suitable methods will be known to a person skilled in the art. For example, the polyclonal antibodies may be produced by immunising a mammal with an immunising antigen to induce the production of antibodies specific for the said immunising antigen. Suitable immunising agents are as defined above. Any suitable mammal may be used such as, for example, mice, rats or rabbits. The polyclonal antibodies produced by the immunised mammal may be obtained by any suitable method. Suitable methods will be known to a person skilled in the art. For example, the polyclonal antibodies may be obtained by collecting the serum of the immunised mammal.

The polyclonal antibodies produced by immunising a mammal with an immunising antigen and collecting the serum of the immunised mammal may be used directly or may be purified. Preferably, the monoclonal antibodies may be purified. The polyclonal antibodies may be purified by any suitable method. Suitable methods will be known to a person skilled in the art. For example, the polyclonal antibodies may be purified by ammonium sulphate precipitation, ion exchange chromatography or an anti-IgG antibody column.

The polyclonal antibodies may be screened for binding to PDE10A using any suitable method. Suitable methods will be known to a person skilled in the art. For example, the polyclonal antibodies may be screened for binding to PDE10A using enzyme immunoassay, such as enzyme-linked immunosorbent assay (ELISA), radioimmunoassay (RIA) or surface plasmon resonance (SPR). Preferably, the polyclonal antibodies may be screened for binding to the PDE10A using enzyme-linked immunosorbent assay (ELISA).

The antibody used in the present invention may be any suitable isotype. Preferably, the antibody is of isotype IgG. For the avoidance of doubt, antibodies of the isotype IgG typically comprise four peptide chains, of which two are heavy chains and two are light chains, and have two fragment antigen-binding (Fab) regions. The Fab regions comprise complementary determining regions (CDRs) which are the part of the antibody which bind to the antigen.

Whilst the inhibitor or inhibitors may comprise a whole antibody or whole antibodies, it may comprise an antibody fragment or a modified form thereof. Suitable examples of whole antibodies include, but are not limited to, monovalent or divalent antibodies. Suitable examples of antibody fragments include, but are not limited to, Fab, F(ab')₂, Fv, Fab/c having one Fab and complete Fc, and single chain Fv (scFv) having heavy (H) or light (L) chain Fvs connected by a suitable linker. Other antibody scaffold proteins may be employed, such as Nanobodies RTM (these constructs, marketed by Ablynx (Belgium), comprise synthetic single immunoglobulin variable heavy domain derived from a camelid (e.g. camel or llama) antibody), Domain Antibodies (marketed by Domantis (Belgium), comprising an affinity matured single immunoglobulin variable heavy domain or immunoglobulin variable light domain), UniBodies (marketed by Genmab, UniBodies are modified fully human IgG4 antibodies where the hinge region of the antibody has been eliminated), Trifunctional Antibodies (monoclonal antibodies with binding sites for two different antigens), Affibodies (marketed by Affibody, Affibodies are based on a 58-amino acid residue protein domain, a three helix bundle domain, derived from one of the IgG-binding domains of staphylococcal protein A), Anticalins (antibody mimetics synthesised from human lipocalins, which can also be formatted as dual targeting proteins, so-called Duocalins) or DARPins (Designed Ankyrin Repeat Proteins) (which are another example of antibody mimetic based on repeat proteins, such as ankyrin or leucine-rich repeat proteins, which are ubiquitous binding molecules).

In certain embodiments, the antibody may be optimised or may be humanised. 'Optimised', and like terms as used herein, means that the amino acid sequence of the antibody is adapted, such as by mutation or modification including, for example, glycosylation, so as to be suitable for use in the patient to which it is to be administered. 'Humanised', and like terms as used herein, means that the amino acid sequence of the antibody is adapted, such as by mutation or modification including, for example, glycosylation, to reduce the composition of non-human amino acid sequences in the antibody.

The antibody, when humanised, may be partially humanised or may be substantially fully humanised. By 'partially humanised' is mean that part of the amino acid sequence of the antibody has been adapted, such as by mutation or modification including, for example, glycosylation, to be the same as the amino acid sequence of the human antibody. The antibody, when partially humanised, may be partially humanised in any region of the antibody. Preferably, the antibody, when partially humanised, may be partially humanised in one or more of the variable fragment antigen-binding (Fab) regions of the antibody. By 'substantially fully humanised' is meant that substantially all of the amino acid sequence of the antibody has been adapted, such as by mutation or modification including, for example, glycosylation, to be the same as the amino acid sequence of the human antibody. Preferably, the antibody is substantially fully humanised.

The optimised and/or humanised antibody and derivatives may be produced by any suitable method. Suitable methods will be known to a person skilled in the art. For example, the optimised and/or humanised antibody and derivatives thereof may be produced using genetic engineering technology, chimaeric technologies, CDR grafting or veneering.

In certain embodiments, when the optimised and/or humanised antibody and derivatives thereof are produced using genetic engineering technology, a polynucleotide encoding the antibody may be isolated and cloned into an expression vector to obtain a recombinant plasmid, transforming a host organism with the obtained recombinant plasmids, culturing the transformants and causing expression of the polynucleotide encoding the antibody. When more than one polynucleotide is used, each polynucleotide may be cloned into the same or different expression vectors. Any suitable host organism may be transformed with the obtained recombinant plasmids. For example, the host organism may be prokaryotic, such as *E.coli, bacilli* including *Bacillus subtilis* and enterobacteriaceae including *Salmonella typhimurium,* or eukaryotic, such as yeast including *Saccharamyces cerevisiae.* It will be appreciated by a person skilled in the art that the step of cloning into an expression vector to produce a recombinant plasmid may be performed using standard methods in the same or different organism to the host organism. Preferably, the step of cloning into an expression vector to produce a recombinant plasmid may be performed using standard methods in *E. coli.*

The optimised and/or humanised antibody may bind to any suitable antigen. Preferably, the optimised and/or humanised antibody binds to PDE10A.

The antibody or antibody mixtures may be present in the composition at any suitable amount. Preferably, the antibody may be present at in an amount of about 0.1 nanograms (ng) to 100 micrograms (mg), more preferably about 1 ng to 50 mg, most preferably about 10 mg to 50 mg.

In other embodiments, the inhibitor or inhibitors comprise a peptide or peptide mimetic thereof, or C-terminal amidated peptide thereof.

The terms "peptide" and "peptides" include compounds that have amino acid residues (H-Cα-[side chain]) but which may be joined by peptide (-CO-NH-) or non-peptide linkages.

Peptides may be synthesised by the Fmoc-polyamide mode of solid-phase peptide synthesis. Reagents for peptide synthesis are readily commercially available.

Purification of the peptides may be effected by any one, or a combination of, techniques such as size exclusion chromatography, ion-exchange chromatography and (principally) reverse-phase high performance liquid chromatography. Analysis of peptides may be carried out using thin layer chromatography, reverse-phase high performance liquid chromatography, amino-acid analysis after acid hydrolysis and by fast atom bombardment (FAB) mass spectrometric analysis.

The peptide may contain at least one D-amino acid residue, such as 1, 2 or 3 or 4 or 5 or 6 or 7 or 8 D-amino acids. Typically, the composition of the invention may contain 0, 1, 2 or 3 D-amino acids. The presence of D-amino acids in the composition of the invention may be useful in preventing degradation of the compound by proteases. Other methods for making peptides resistant to proteolytic degradation include blocking the N- and/or C- terminal amino acid residues. Thus, in some embodiments the N- and/or C-terminal amino acid residues are blocked. Suitable blocking methods include acetylation of the N-terminus or incorporating a pyroglutamate residue at the N-terminus.

The peptide may be a peptide aptamer. Peptide aptamers typically consist of short, 5-20 amino acid residues long sequences that can bind to a specific target molecule.

There are a number of different approaches to the design and synthesis of peptide composition that do not contain amide bonds. In one approach, one or more amide bonds are replaced in an essentially isoteric manner by a variety of chemical functional groups.

Retro-inverso peptidomimetics, in which the peptide bonds are reversed, can be synthesised by methods known in the art. This approach involves making pseudopeptides containing changes involving the backbone, and not the orientation of side chains. Retro-inverse peptides, which contain NH-CO bonds instead of CO-NH peptide bonds, are more resistant to proteolysis.

The peptide may be linear. Although, it may be advantageous to introduce a cyclic moiety into a peptide-based framework. The cyclic moiety restricts the conformational space of the peptide structure and this may lead to an increased efficacy. An added advantage of this strategy is that the introduction of a cyclic moiety into a peptide may also result in the peptide having a diminished sensitivity to cellular peptidases.

In some embodiments of the invention the peptide may be joined to another moiety. Convenient moieties to which the peptide may be joined include polyethylene glycol (PEG) and peptide sequences, such as TAT and antennapedia which enhance delivery to cells.

PEGylation is a method well known to those skilled in the art wherein a (peptide or other compound) is modified such that one or more polyethylene glycol (PEG) molecules are covalently attached to the side chain of one or more amino acids. It is one of the most important molecule altering structural chemistry techniques (MASC). Other MASC techniques may be used; such techniques may improve the pharmacodynamic properties of a compound, for example extending its serum half-life *in vivo.* A PEG-peptide conjugate is formed by first activating the PEG moiety so that it will react with, and couple to, the compound of the invention. PEG moieties vary considerably in molecular weight and conformation, with the early moieties (monofunctional PEGs; mPEGs) being linear with molecular weights of 12kDa or less, and later moieties being of increased molecular weights. PEG2, a recent innovation in PEG technology, involves the coupling of a 30kDa (or less) mPEG to a lysine amino acid (although PEGylation can be extended to the addition of PEG to other amino acids) that is further reacted to form a branched structure that behaves like a linear mPEG of much greater molecular weight. Methods that may be used to covalently attach the PEG molecules to the peptides. The potential advantages of PEGylation of the compound of the invention include reduced renal clearance which, for some products, results in a more sustained adsorption after subcutaneous administration as well as restricted distribution, possibly leading to a more constant and sustained plasma concentrations and hence an increase in clinical effectiveness. Further potential advantages include reduced immunogenicity of the therapeutic compound, and lower toxicity.

In some embodiments, the inhibitor or inhibitors is/are pro-drugs of the peptide. A pro-drug is a compound which is metabolised *in vivo* to produce the molecule, such as a protein. One of skill in the art will be familiar with the preparation of pro-drugs.

The peptide may be a peptide mimetic. A peptide mimetic is an organic compound having similar geometry and polarity to the molecules defined herein, and which has a substantially similar function. A mimetic may be a molecule in which the NH groups of one or more peptide links are replaced by CH₂ groups. A mimetic may be a molecule in which one or more amino acid residues is replaced by an aryl group, such as a napthyl group.

In other embodiments, an inhibitor or inhibitors comprise nucleic acid, such as single stranded DNA or RNA, which is capable of binding to and inhibiting PDE10A. It is envisaged that the same targets on PDE10A are also suitable for targeting with peptides and peptide aptamers will also be suitable for targeting with RNA or modified RNA aptamers. Nucleic acids such as single stranded DNAs and RNAs may be provided that bind to and inhibit PDE10A expression by binding to mRNA or DNA. Typically, the nucleic acids are single stranded and have from 100 to 5000 bases.

Features, integers, characteristics, compounds, molecules, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and figures), and/or all of the steps of any method or process so disclosed, may be combined in any combination, except combinations where at least some of such features and/or steps are mutually exclusive.

### Detailed Description of the Invention

Embodiments of the invention are described below, by way of example only, with reference to the accompanying figures in which:
Figure 1 are plots showing RNA expression of PDE10A in normal tissue. The plots represent baseline gene expression of PDE10A and GUCY2C (guanylate cyclase 2C) in healthy samples based on GTEx data, where the X-axis represents tissue, y-axis represents log₂ transformed expression;

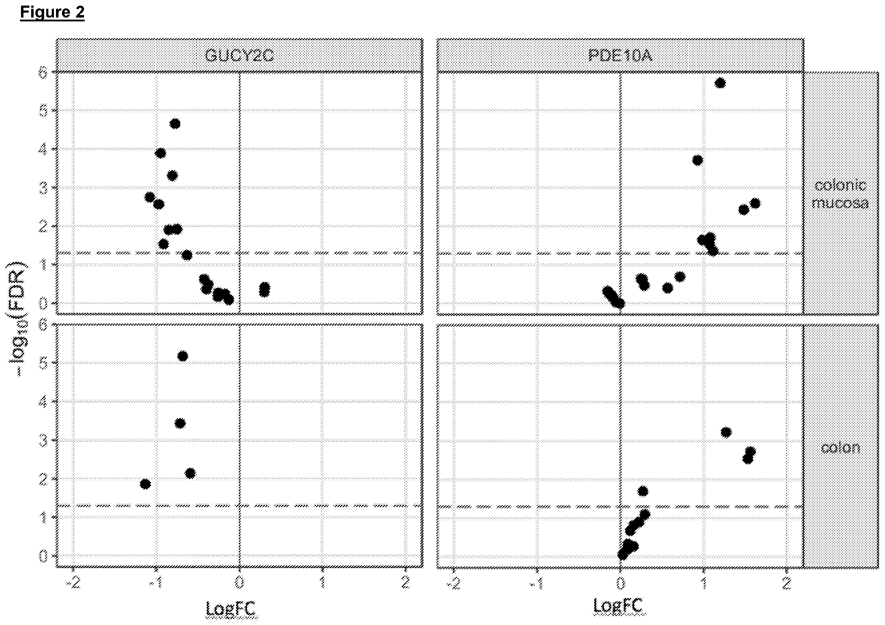

Figure 2 are volcano plots showing differential RNA expression of PDE10A and GUCY2C. The volcano plots show differential gene expression for a selected comparison, where the x-axis represents log fold change (FC) and y-axis represents log₁₀ transformed adjusted p-value (FDR). Horizontal dotted line is FDR=0.05 threshold and values above the dotted line are considered significant. Values to the right of the central axis indicate upregulation, values to the left of the central axis indicate down regulation. The OmicSoft differential expression datasets used in the analysis were as follows: colonic mucosa -OmicSoft Project names: GSE14580, GSE16879, GSE36807, GSE59071, GSE65114, GSE73661; colon - OmicSoft Project names: GSE10191, GSE10616, GSE6731, GSE9686;
Figure 3 is a graph showing the effect of a PDE10A inhibitor PF-02545920 on isolated human neutrophil activation in response to IL-8;
Figure 4 are graphs showing PF-02545920 and TAK-063 inhibiting the release of inflammatory cytokines IL-6 and IL-8 in ex-vivo cultures of colon biopsy samples from UC patients. A. UC donor 1, B. UC donor 2 (n=2; Mean±SD), wherein Pred = prednisolone, Tofa = tofacitinib;
Figure 5 are graphs showing PF-02545920 and TAK-063 inhibiting the release of inflammatory cytokines IL-6 and IL-8 in ex-vivo cultures of inflamed UC tissue. A. UC donor 1, B. UC donor 2 (n=5; Mean±SD; * p<0.05), wherein Pred = prednisolone, Tofa = tofacitinib ; and
Figure 6 are graphs showing PF-02545920 (1µM) inhibiting release of the inflammatory cytokine TNFα in ex-vivo cultures of inflamed colon tissue obtained from surgical resection from treatment-refractory UC patients. A. UC donor 1, B. UC donor 2 (n=5; Mean±SD; * p<0.05).

### Example 1

### Assessing a PDE10A inhibitor for use in the treatment of ulcerative colitis

To explore the role of PDE 10A in ulcerative colitis (UC) the Genotype-Tissue Expression (GTEx) database was used to look at PDE10A RNA expression in normal and diseased tissues. Alongside this expression levels of guanylate cyclase 2C (GUCY2C) were also assessed. GUCY2C is an enzyme which synthesises cGMP in response to the endogenous peptides guanylin and uroguanylin as well as *E.coli* heat-stable enterotoxin.

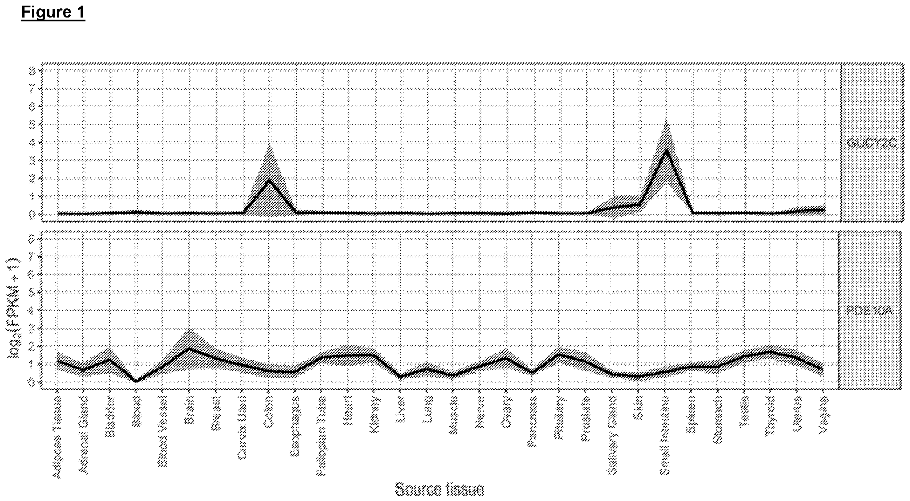

As previously described in the literature, in normal tissue PDE10A is expressed at low levels except in brain (as shown in Figure 1). However in colonic mucosa and colon tissue from ulcerative colitis patients, PDE10A expression levels were significantly upregulated compared to normal controls (as shown in Figure 2). This is a finding that has not been previously described in the literature and highlighting a potential undiscovered role for PDE10A in UC pathology.

GUCY2C was seen to be specifically expressed at high levels in colon and small intestine (as shown in Figure 1) suggesting a role for this enzyme in normal gut homeostasis. In UC colonic mucosa and colon, GUCY2C was significantly downregulated (as shown in Figure 2), a finding that has previously been described in the literature.

Guanylate cyclase-C and cGMP signalling is downregulated in ulcerative colitis (Brenna et al The guanylate cyclase-C signaling pathway is down-regulated in inflammatory bowel disease Scand J Gastroenterol. 2015;50(10):1241-52) and decreases in expression of guanylate cyclase 2C, guanylin, and uroguanylin correlate with severity of disease. (Lan et al. Expression of guanylate cyclase-C, guanylin, and uroguanylin is downregulated proportionally to the ulcerative colitis disease activity index Sci Rep. 2016; 6: 25034. Published online 2016 Apr 29. doi: 10.1038/srep25034)*.* This suggests that reduced cGMP signalling plays a role in UC pathology. cGMP in the GI tract has also been shown to play a role in fluid and electrolyte secretion, barrier function, inflammation and proliferation (Waldman et al, Guanylate cyclase-C as a therapeutic target in gastrointestinal disorders., Gut. 2018 67(8):1543-1552)*.*

While less studied in inflammation than cAMP, reduced cGMP signalling has also been shown to increase inflammation in other systems (Ahluwalia et al, Antiinflammatory activity of soluble guanylate cyclase: cGMP-dependent down-regulation of P-selectin expression and leukocyte recruitment. Proc Natl Acad Sci U S A. 2004 101(5): 1386-91*;* Rapôso et al, Role of iNOS-NO-cGMP signaling in modulation of inflammatory and myelination processes.Brain Res Bull. 2014 104:60-73)

Taken together, in UC colon and colonic mucosa, cGMP hydrolysing activity by PDE10A would be increased and cGMP synthesizing activity by guanylate cyclase 2C would be decreased resulting in a net decrease in cGMP levels and signalling.

Experiments were then conducted to assess whether inhibiting PDE10A with a small molecule inhibitor would help restore cGMP signalling to normal levels and therefore represent a useful treatment for ulcerative colitis.

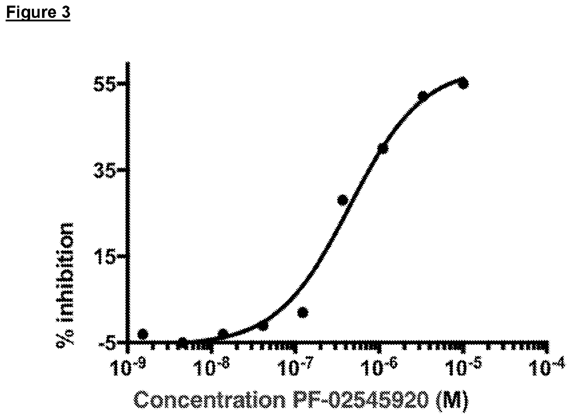

A PDE10A selective tool compound PF-02545920 was assessed in an *in vitro* assay of IL-8 neutrophil activation. PF-02545920 dose dependently inhibited IL-8 induced neutrophil activation (as shown in Figure 3). This was of interest as a role for PDE10A in neutrophil function had not been previously described and further suggested a role for PDE10A in modulating inflammation and that a PDE10A inhibitor would be suitable as a therapeutic for inflammatory bowel diseases, and ulcerative colitis in particular.

The therapeutic potential of inhibitors of PDE10A to treat ulcerative colitis was further assessed using tissue samples from inflamed colonic mucosa from ulcerative colitis patients.

The effect of selective PDE10 inhibition was tested on inflamed colonic mucosa from ulcerative colitis patients taken during routine endoscopy (Method 1 detailed below). These samples retain a disease phenotype in *ex-vivo* culture and secrete high basal levels of inflammatory cytokines. The effect of selective PDE10 inhibition on levels of the inflammatory cytokines IL-8 and IL-6 released from these tissue samples was measured. Both IL-6 and IL-8 are key regulators in ulcerative colitis pathology and their levels correlate with disease severity (Waldner MJ et al., Master regulator of intestinal disease: IL-6 in chronic inflammation and cancer development. Semin Immunol. 2014 26(1),75-9.; Bernardo D et al, IL-6 promotes immune responses in human ulcerative colitis and induces a skin-homing phenotype in the dendritic cells and T-cells they stimulate. Eur J Immunol. 2012, 42(5),1337-53.; Pearl DS, Cytokine mucosal expression in ulcerative colitis, the relationship between cytokine release and disease activity. J Crohns Colitis. 2013, 7(6), 481-9).

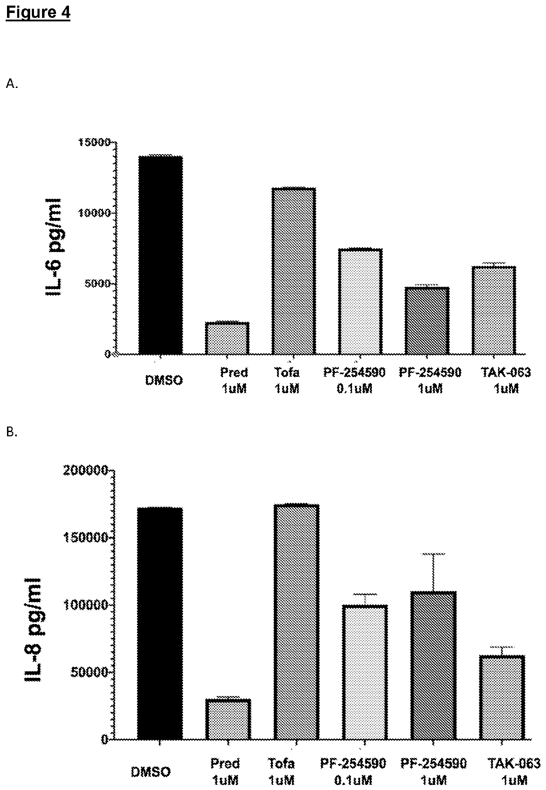

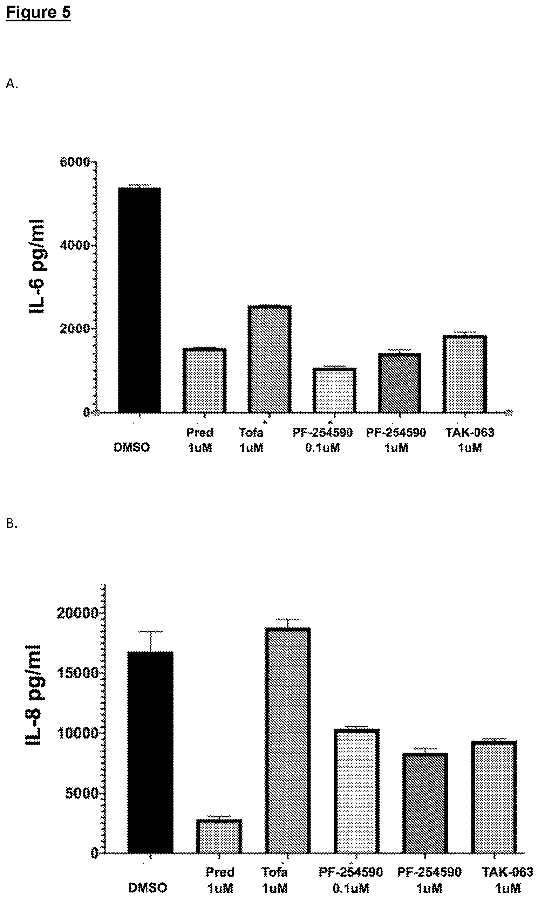

The structurally distinct PDE10A inhibitors PF-02545920 and TAK-063 were tested alongside 2 positive control compounds, the steroid prednisolone and the Janus kinase inhibitor tofacitinib, in colon biopsy samples from two ulcerative colitis patients. These colon biopsies retain an inflammatory phenotype and secrete high levels of inflammatory cytokines in ex-vivo culture. Selective PDE10A inhibition significantly reduced the secreted levels of IL-6 and IL-8 when compared to the DMSO vehicle (Figures 4 and 5) This reduction was comparable to that seen with the positive controls. PF-02545920 was tested at concentrations of 0.1µM and 1µM. The doses tested of each inhibitor will result in selective PDE10A inhibition over the other PDE family members.

In isolated enzyme biochemical assays, PF-02545920 has been shown to be a highly selective PDE10A inhibitor with an IC50 for PDE10A <5nM and IC50s for other PDE family members >1 µM (Grauer SM et.al. Phosphodiesterase 10A inhibitor activity in preclinical models of the positive, cognitive, and negative symptoms of schizophrenia. J Pharmacol Exp Ther. 2009 331(2), 574-90.) Therefore, at the test concentration of 0.1µM and 1µM in an ex-vivo tissue assay, PF-02545920 will selectively inhibit PDE10A.

In isolated enzyme biochemical assays, TAK-063 has been shown to be a highly selective PDE10A inhibitor with an IC50 for PDE10A of 0.3nM and IC50s for other PDE family members >5 µM (Kunitomo J et.al. Discovery of 1-[2-fluoro-4-(1H-pyrazol-1-yl)phenyl]-5-methoxy-3-(1-phenyl-1H-pyrazol-5-yl)pyridazin-4(1H)-one (TAK-063), a highly potent, selective, and orally active phosphodiesterase 10A (PDE10A) inhibitor. J Med.Chem. 2014 57(22),9627-43.) Therefore, at the test concentration of 1uM in an ex-vivo tissue assay, TAK-063 will selectively inhibit PDE10A.

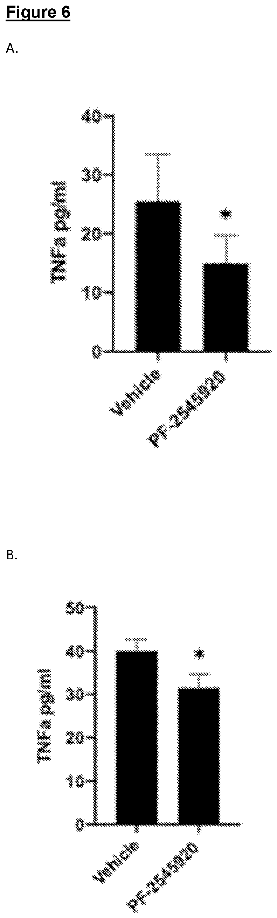

The effect of selective PDE10A inhibition was also tested on inflamed colonic mucosa from pharmacotherapy treatment-refractory ulcerative colitis patients taken during colon resection surgery (Method 2 detailed below). The PDE10A inhibitor PF-02545920 (1 µM) was tested in colon samples from two ulcerative colitis patients. The effect of selective PDE10A inhibition on levels of the inflammatory cytokine TNFα released from these tissue samples were measured. TNFα is a pro-inflammatory mediator that is expressed at high levels in the colonic mucosa of patients with UC and is the target of anti-TNFα biologics which have demonstrated efficacy in the treatment of UC (Pugliese D. et al, Anti TNF-α therapy for ulcerative colitis: current status and prospects for the future., Expert Rev Clin Immunol. 2017 13(3):223-233.). Selective PDE10A inhibition significantly reduced the secreted levels of TNFα compared to the DMSO vehicle (Figure 6.)

The ability of selective PDE10A inhibition to significantly reduce levels of inflammatory cytokines in UC patient-derived colonic mucosa demonstrates the therapeutic utility of PDE10A inhibitors for the treatment of UC.

### Method 1

Biopsy tissue was obtained from inflamed colonic mucosa from ulcerative colitis patients during routine endoscopy. Ex-vivo biopsy cultures for the analysis of inflammatory cytokine biomarkers were run as previously described (Vossenkämper A. et al. A CD3-specific antibody reduces cytokine production and alters phosphoprotein profiles in intestinal tissues from patients with inflammatory bowel disease. Gastroenterology, 2014, 147, 172-183). Biopsies were incubated in organ culture for 24 h with the addition of positive control compounds, or the specific PDE10A inhibitor PF-02545920. Supernatants collected at the end of the experiment were snap-frozen and stored at -70 °C. For the measurement of cytokines, the frozen culture supernatants were thawed and analysed for levels of the inflammatory cytokines using Luminex cytokine assay kits (R&D Systems) and an R&D Systems MAGPIX^{®} analyser. Mean values ±SDs were calculated for the levels of spontaneous cytokine production measured in biopsy culture supernatants from each treatment group.

### Method 2

Ulcerative colitis donor samples were obtained with full ethical consent from patients undergoing therapeutic resection for ulcerative colitis. Tissues were placed apical (mucosal) side facing upwards on a Netwell filter. The biopsies were then be cultured in either control media or media containing the test compound in an incubator at 37 °C and high O₂ atmospheric conditions. To try to minimize variation, the biopsies were also cultured in the presence of the inflammatory stimulant Staphylococcal Enterotoxin B (SEB) to help normalise cytokine levels. At approximately 18 hours post-culture start, media samples were collected, protease inhibitor added and samples stored at -80 °C. Supernatants were subsequently subjected to ELISA analysis for cytokine measurement.

### Example 2

### Example Formulations and Treatments for Ulcerative Colitis

A number of example formulations are provided below along with suggested dosage regimes. It will be understood that these are for illustrative purposes and these would be optimized during further experimentation, which may include clinical trials. For simplicity, the formulations do not stipulate any non-active components (such as pharmaceutically acceptable carriers or excipients etc.)

### Formulation 2A - TAK-063 - Oral Tablet for the Treatment of Ulcerative Colitis

| **Active Ingredient** | **Form** | **mg** | **Dose** |
|---|---|---|---|
| TAK-063 | Oral Tablet | 20 | Once daily |

### Formulation 2B - TAK-063 - Oral Tablet for the Treatment of Ulcerative Colitis

| **Active Ingredient** | **Form** | **mg** | **Dose** |
|---|---|---|---|
| TAK-063 | Oral Tablet | 10 | Once daily |

### Formulation 2C - Papaverine - Oral Tablet for the Treatment of Ulcerative Colitis

| **Active Ingredient** | **Form** | **mg** | **Dose** |
|---|---|---|---|
| Papaverine | Oral Tablet | 150 | Twice daily |

### Formulation 2D - PF-02545920 - Oral Tablet for the Treatment of Ulcerative Colitis

| **Active Ingredient** | **Form** | **mg** | **Dose** |
|---|---|---|---|
| PF-02545920 | Oral Tablet | 20 | Twice daily |

### Formulation 2E - PF-02545920 - Oral Tablet for the Treatment of Ulcerative Colitis

| **Active Ingredient** | **Form** | **mg** | **Dose** |
|---|---|---|---|
| PF-02545920 | Oral Tablet | 5 | Twice daily |

### Formulation 2F - PQ-10 - Intravenous Injection for the Treatment of Ulcerative Colitis

| **Active Ingredient** | **Form** | **mg/kg** | **Dose** |
|---|---|---|---|
| PQ-10 | Intravenous injection | 0.1 | Once daily |

### Formulation 2G - PQ-10 - Intravenous Injection for the Treatment of Ulcerative Colitis

| **Active Ingredient** | **Form** | **mg/kg** | **Dose** |
|---|---|---|---|
| PQ-10 | Intravenous injection | 3 | Once daily |

### Formulation 2H - PDM-042 - Intravenous Injection for the Treatment of Ulcerative Colitis

| **Active Ingredient** | **Form** | **mg/kg** | **Dose** |
|---|---|---|---|
| PDM-042 | Intravenous injection | 0.1 | Once daily |

### Formulation 2I - PDM-042 - Intravenous Injection for the Treatment of Ulcerative Colitis

| **Active Ingredient** | **Form** | **mg/kg** | **Dose** |
|---|---|---|---|
| PDM-042 | Intravenous injection | 3 | Once daily |

The skilled addressee will of course understand that alternative PDE10A inhibitors could also be employed in place of those outlined above. The therapeutically effective doses will of course depend on the activity and format of the chosen inhibitor.

## Claims

1. A composition comprising a PDE10A inhibitor for use in the prevention, management and/or treatment of inflammatory bowel diseases.

2. The composition for use according to claim 1, wherein the inflammatory bowel disease is ulcerative colitis.

3. The composition for use according to either claim 1 or 2, wherein the PDE10A inhibitor is a selective inhibitor of PDE10A.

4. The composition for use according to any preceding claim, wherein the PDE10A inhibitor is selected from one or more of the following: PF-02545920, TAK-063, Papaverine, JNJ-42314415, AMG-579, PQ-10, BMS-843496 and PDM-042.

5. The composition for use according to any preceding claim, wherein the PDE10A inhibitor is selected from PF-02545920 or TAK-063.

6. The composition for use according to any preceding claim, wherein the PDE10A inhibitor selectively inhibits cGMP hydrolysis over cAMP hydrolysis.

7. The composition for use according to any preceding claim, wherein the management and/or treatment of inflammatory bowel diseases of the present invention involves increasing cGMP signalling in bowel tissue of a patient.

8. The composition for use according to any preceding claim, wherein the management and/or treatment of inflammatory bowel diseases of the present invention involves reducing levels of inflammatory cytokines in bowel tissue of a patient.

9. An inhibitor of PDE10A for use in the prevention, management and/or treatment of inflammatory bowel diseases.

10. The inhibitor for use according to claim 9, wherein the inflammatory bowel disease is ulcerative colitis.

11. The inhibitor for use according to either claim 9 or 10, wherein the PDE10A inhibitor is a selective inhibitor of PDE10A.

12. The inhibitor for use according to any one of claims 9 to 11, wherein the PDE10A inhibitor is selected from one or more of the following: PF-02545920, TAK-063, Papaverine, JNJ-42314415, AMG-579, PQ-10, BMS-843496 and PDM-042.

## Patentansprüche

1. Zusammensetzung, die einen PDE10A-Inhibitor zur Verwendung bei der Vorbeugung, dem Management und/oder der Behandlung von entzündlichen Darmerkrankungen umfasst.

2. Zusammensetzung zur Verwendung nach Anspruch 1, wobei es sich bei der entzündlichen Darmerkrankung um Colitis ulcerosa handelt.

3. Zusammensetzung zur Verwendung nach einem der Ansprüche 1 oder 2, wobei es sich bei dem PDE10A-Inhibitor um einen selektiven Inhibitor von PDE10A handelt.

4. Zusammensetzung zur Verwendung nach einem der vorstehenden Ansprüche, wobei der PDE10A-Inhibitor aus einer oder mehreren der folgenden Substanzen ausgewählt ist: PF-02545920, TAK-063, Papaverin, JNJ-42314415, AMG-579, PQ-10, BMS-843496 und PDM-042.

5. Zusammensetzung zur Verwendung nach einem der vorstehenden Ansprüche, wobei der PDE10A-Inhibitor aus PF-02545920 oder TAK-063 ausgewählt ist.

6. Zusammensetzung zur Verwendung nach einem der vorstehenden Ansprüche, wobei der PDE10A-Inhibitor selektiv die cGMP-Hydrolyse gegenüber der cAMP-Hydrolyse hemmt.

7. Zusammensetzung zur Verwendung nach einem der vorstehenden Ansprüche, wobei das Management und/oder die Behandlung von entzündlichen Darmerkrankungen der vorliegenden Erfindung die Erhöhung der cGMP-Signalisierung im Darmgewebe eines Patienten umfassen.

8. Zusammensetzung zur Verwendung nach einem der vorstehenden Ansprüche, wobei das Management und/oder die Behandlung von entzündlichen Darmerkrankungen der vorliegenden Erfindung die Verringerung der Konzentration von entzündlichen Zytokinen im Darmgewebe eines Patienten umfassen.

9. PDE10A-Inhibitor zur Verwendung bei der Vorbeugung, dem Management und/oder der Behandlung von entzündlichen Darmerkrankungen.

10. Inhibitor zur Verwendung nach Anspruch 9, wobei es sich bei der entzündlichen Darmerkrankung um Colitis ulcerosa handelt.

11. Inhibitor zur Verwendung nach einem der Ansprüche 9 oder 10, wobei es sich bei dem PDE10A-Inhibitor um einen selektiven Inhibitor von PDE10A handelt.

12. Inhibitor zur Verwendung nach einem der Ansprüche 9 bis 11, wobei der PDE10A-Inhibitor aus einer oder mehreren der folgenden Substanzen ausgewählt ist: PF-02545920, TAK-063, Papaverin, JNJ-42314415, AMG-579, PQ-10, BMS-843496 und PDM-042.

## Revendications

1. Composition comprenant un inhibiteur de PDE10A pour une utilisation dans la prévention, la gestion et/ou le traitement de maladies intestinales inflammatoires.

2. Composition pour une utilisation selon la revendication 1, la maladie intestinale inflammatoire étant la colite ulcéreuse.

3. Composition pour une utilisation selon l'une ou l'autre parmi la revendication 1 ou 2, l'inhibiteur de PDE10A étant un inhibiteur sélectif de PDE10A.

4. Composition pour une utilisation selon une quelconque revendication précédente, l'inhibiteur de PDE10A étant choisi parmi l'un ou plusieurs parmi les suivants : PF-02545920, TAK-063, Papavérine, JNJ-42314415, AMG-579, PQ-10, BMS-843496 et PDM-042.

5. Composition pour une utilisation selon une quelconque revendication précédente, l'inhibiteur PDE10A étant choisi parmi PF-02545920 ou TAK-063.

6. Composition pour une utilisation selon une quelconque revendication précédente, l'inhibiteur PDE10A inhibant sélectivement l'hydrolyse de cGMP par rapport à l'hydrolyse de cAMP.

7. Composition pour une utilisation selon une quelconque revendication précédente, la gestion et/ou le traitement de maladies intestinales inflammatoires de la présente invention impliquant une augmentation de la signalisation de cGMP dans un tissu intestinal d'un patient.

8. Composition pour une utilisation selon une quelconque revendication précédente, la gestion et/ou le traitement de maladies intestinales inflammatoires de la présente invention impliquant une réduction des taux de cytokines inflammatoires dans un tissu intestinal d'un patient.

9. Inhibiteur de PDE10A pour une utilisation dans la prévention, la gestion et/ou le traitement de maladies intestinales inflammatoires.

10. Inhibiteur pour une utilisation selon la revendication 9, la maladie intestinale inflammatoire étant la colite ulcéreuse.

11. Inhibiteur pour une utilisation selon l'une ou l'autre parmi la revendication 9 ou la revendication 10, l'inhibiteur de PDE10A étant un inhibiteur sélectif de PDE10A.

12. inhibiteur pour une utilisation selon l'une quelconque des revendications 9 à 11, l'inhibiteur de PDE10A étant choisi parmi l'un ou plusieurs parmi les suivants : PF-02545920, TAK-063, Papavérine, JNJ-42314415, AMG-579, PQ-10, BMS-843496 et PDM-042.
